(19) Europäisches Patentamt
European Patent Office
Office européen des brevets

(11) **EP 3 360 557 A2**

(12) **EUROPEAN PATENT APPLICATION**

(43) Date of publication:
**15.08.2018 Bulletin 2018/33**

(51) Int Cl.:
*A61K 33/00* (2006.01)     *A61P 11/00* (2006.01)
*A61P 9/12* (2006.01)     *A01N 59/00* (2006.01)

(21) Application number: **17206023.8**

(22) Date of filing: **02.12.2011**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **03.12.2010 US 419657 P**

(62) Document number(s) of the earlier application(s) in accordance with Art. 76 EPC:
**11844660.8 / 2 645 860**

(71) Applicant: **Geno LLC**
**Cocoa, FL 32926 (US)**

(72) Inventors:
• **FINE, David H.**
**Cocoa Beach, FL 32932 (US)**
• **ROSCIGNO, Robert F**
**Cocoa, FL 32926 (US)**

• **VASQUEZ, Gregory**
**Cocoa, FL 32922 (US)**
• **JOHNSON, Bryan**
**Orlando, FL 32822 (US)**
• **DENTON, Ryan**
**Titusville, FL 32780 (US)**

(74) Representative: **D Young & Co LLP**
**120 Holborn**
**London EC1N 2DY (GB)**

Remarks:
•Claims filed after the date of filing of the application/after the date of receipt of the divisional application (Rule 68(4) EPC)
•This application was filed on 07-12-2017 as a divisional application to the application mentioned under INID code 62.

(54) **NITRIC OXIDE TREATMENTS**

(57) Portable and pure nitric oxide delivery systems can be used to treat a variety of patient conditions.

## Description

### PARA OF PRIORITY

This application paras the benefit of prior U.S. Provisional Application No. 61/419,657 filed on December 3, 2010, which is incorporated by reference in its entirety.

### TECHNICAL FIELD

This invention relates to nitric oxide treatments.

### BACKGROUND

Nitric oxide (NO), also known as nitrosyl radical, is a free radical that is an important signalling molecule. For example, NO can cause smooth muscles in blood vessels to relax, thereby resulting in vasodilation and increased blood flow through the blood vessel. These effects can be limited to small biological regions since NO can be highly reactive with a lifetime of a few seconds and can be quickly metabolized in the body.

Some disorders or physiological conditions can be mediated by inhalation of nitric oxide. The use of low concentrations of inhaled nitric oxide (NO) can prevent, reverse, or limit the progression of disorders which can include, but are not limited to, acute pulmonary vasoconstriction, traumatic injury, aspiration or inhalation injury, fat embolism in the lung, acidosis, inflammation of the lung, adult respiratory distress syndrome, acute pulmonary edema, acute mountain sickness, post cardiac surgery acute pulmonary hypertension, persistent pulmonary hypertension of a newborn, perinatal aspiration syndrome, haline membrane disease, acute pulmonary thromboembolism, heparin-protamine reactions, sepsis, asthma and status asthmaticus or hypoxia. Nitric oxide (NO) can also be used to treat chronic pulmonary hypertension, bronchopulmonary dysplasia, chronic pulmonary thromboembolism and idiopathic or primary pulmonary hypertension or chronic hypoxia. Typically, the NO gas is supplied in a bottled gaseous form diluted in nitrogen gas ($N_2$). Great care has to be taken to prevent the presence of even trace amounts of oxygen ($O_2$) in the tank of NO gas because the NO, in the presence of $O_2$, is oxidized to nitrogen dioxide ($NO_2$). Unlike NO, the part per million levels of $NO_2$ gas is highly toxic if inhaled and can form nitric and nitrous acid in the lungs.

Generally, nitric oxide can be inhaled or otherwise delivered to the individual's lungs. Providing a therapeutic dose of NO could treat a patient suffering from a disorder or physiological condition that can be mediated by inhalation of NO or supplement or minimize the need for traditional treatments in such disorders or physiological conditions. Typically, the NO gas can be supplied in a bottled gaseous form diluted in nitrogen gas ($N_2$). Great care should be taken to prevent the presence of even trace amounts of oxygen ($O_2$) in the tank of NO gas because the NO, in the presence of $O_2$, can be oxidized to nitrogen dioxide ($NO_2$). Unlike NO, the part per million levels of $NO_2$ gas can be highly toxic if inhaled and can form nitric and nitrous acid in the lungs.

### SUMMARY

Portable and pure nitric oxide delivery systems can be used to treat a variety of patient conditions.

In one aspect, a method of treating a patient after cardiopulmonary resuscitation has been performed on the patient can include passing nitrogen dioxide through a cartridge configured to convert the nitrogen dioxide into nitric oxide, and delivering an effective concentration of the nitric oxide to the patient.

In some embodiments, nitric oxide can be delivered to a patient between about 15 minutes and about 3 hours, between about 30 minutes and 2 hours or between about 45 minutes and about 1.25 hours after cardiopulmonary resuscitation has been performed on the patient.

In some embodiments, nitric oxide can be delivered to a patient between at least 15 minutes, at least 30 minutes, at least 45 minutes, at least 1 hour, at least 2 hours, at least 3 hours, at least 4 hours or at least 6 hours after cardiopulmonary resuscitation has been performed on the patient.

In some embodiments, nitric oxide can be delivered to a patient between at most 6 hours, at most 4 hours, at most 3 hours, at most 2 hours, at most 1.5 hours, at most 1.25 hours or at most 1 hour after cardiopulmonary resuscitation has been performed on the patient.

In another aspect, a method of treating a patient after the patient has experienced an event resulting in inflammation in the central nervous system can include passing nitrogen dioxide through a cartridge configured to convert the nitrogen dioxide into nitric oxide, and delivering an effective concentration of the nitric oxide to the patient. In some embodiments, an event resulting in inflammation in the central nervous system can be a stroke or spinal cord injury.

In some embodiments, nitric oxide can be delivered to a patient between about 15 minutes and about 3 hours, between about 30 minutes and 2 hours or between about 45 minutes and about 1.25 hours after the patient has experienced an event resulting in inflammation in the central nervous system.

In some embodiments, nitric oxide can be delivered to a patient between at least 15 minutes, at least 30 minutes, at least 45 minutes, at least 1 hour, at least 2 hours, at least 3 hours, at least 4 hours or at least 6 hours after the patient has experienced an event resulting in inflammation in the central nervous system.

In some embodiments, nitric oxide can be delivered to a patient between at most 6 hours, at most 4 hours, at most 3 hours, at most 2 hours, at most 1.5 hours, at most 1.25 hours or at most 1 hour after the patient has experienced an event resulting in inflammation in the central nervous system.

In another aspect, a method of treating a patient after inflammation resulting from a trauma to the central nervous system has been diagnosed can include passing nitrogen dioxide through a cartridge configured to convert the nitrogen dioxide into nitric oxide, and delivering an effective concentration of the nitric oxide to the patient. In some embodiments, a trauma to the central nervous system can be a stroke or spinal cord injury.

In some embodiments, nitric oxide can be delivered to a patient between about 15 minutes and about 3 hours, between about 30 minutes and 2 hours or between about 45 minutes and about 1.25 hours after inflammation resulting from a trauma to the central nervous system has been diagnosed.

In some embodiments, nitric oxide can be delivered to a patient between at least 15 minutes, at least 30 minutes, at least 45 minutes, at least 1 hour, at least 2 hours, at least 3 hours, at least 4 hours or at least 6 hours after inflammation resulting from a trauma to the central nervous system has been diagnosed.

In some embodiments, nitric oxide can be delivered to a patient between at most 6 hours, at most 4 hours, at most 3 hours, at most 2 hours, at most 1.5 hours, at most 1.25 hours or at most 1 hour after inflammation resulting from a trauma to the central nervous system has been diagnosed.

In another aspect, a method of treating a patient having sleep apnea can include passing nitrogen dioxide through a system configured to convert the nitrogen dioxide into nitric oxide, and delivering an effective concentration of the nitric oxide to the patient. In some embodiments, delivering nitric oxide can include supplying forced air into the patient.

In another aspect, a method of treating a patient having pulmonary arterial hypertension can include passing nitrogen dioxide through a system configured to convert the nitrogen dioxide into nitric oxide, and delivering an effective concentration of the nitric oxide to the patient. In some embodiments, a method can further include delivering a PDE5 inhibitor to the patient.

In another aspect, a method of treating a patient having a pulmonary disorder can include passing nitrogen dioxide through a system configured to convert the nitrogen dioxide into nitric oxide, and delivering an effective concentration of the nitric oxide to the patient.

In some embodiments, the pulmonary disorder can be pulmonary hypertension, chronic obstructive pulmonary disease, idiopathic pulmonary fibrosis, acute chest syndrome, infectious lung disease, hypoxemia, respiratory failure, respiratory distress syndrome, pulmonary embolism, cystic fibrosis, or combinations thereof.

In another aspect, a method of treating a patient having a cardiac or blood disorder can include passing nitrogen dioxide through a system configured to convert the nitrogen dioxide into nitric oxide, and delivering an effective concentration of the nitric oxide to the patient. In some embodiments, the blood disorder can be a sickle cell related disorder. In some embodiments, the cardiac disorder can be heart failure or cardiovascular shock.

In another aspect, a method of treating a patient having a kidney disorder can include passing nitrogen dioxide through a system configured to convert the nitrogen dioxide into nitric oxide, and delivering an effective concentration of the nitric oxide to the patient. In some embodiments, the kidney disorder can include an alpha-1-adrenoreceptor and vasoreactivity.

In another aspect, a method of treating a patient who is attempting to quit smoking can include passing nitrogen dioxide through a system configured to convert the nitrogen dioxide into nitric oxide, and delivering an effective concentration of the nitric oxide to the patient.

In another aspect, a method of treating a patient having a neurologic disorder can include passing nitrogen dioxide through a system configured to convert the nitrogen dioxide into nitric oxide, and delivering an effective concentration of the nitric oxide to the patient.

In another aspect, a method of treating a patient can include passing nitrogen dioxide through a system configured to convert the nitrogen dioxide into nitric oxide, and delivering an effective concentration of the nitric oxide to the patient.

In some embodiments, the patient can be postoperative. In some embodiments, the postoperative patient can have experienced pulmonary or cardiac stress.

In some embodiments, the patient can have experienced ischemic injury or reperfusion injury.

In some embodiments, the patient can have experienced organ failure.

In some embodiments, the patient can have altitude illness or pulmonary edema.

In another aspect, a method of treating a patient having a wound can include passing nitrogen dioxide through a system configured to convert the nitrogen dioxide into nitric oxide, and exposing the wound to an effective concentration of the nitric oxide.

In some embodiments, a cartridge can include a surface-activated material. A surface-activated material can include silica gel or cotton.

In some embodiments, a cartridge can include a reducing agent. Any appropriate reducing agent that can convert $NO_2$ or $N_2O_4$ to NO can be used as determined by a person of skill in the art. For example, reducing agents can include hydroquinones, glutathione, reduced metal salts such as Fe(II), Mo(VI), NaI, Ti(III) or Cr(III), thiols, or $NO_2^-$. The reducing agent can be an antioxidant. The antioxidant can be an aqueous solution of an antioxidant. The antioxidant can be ascorbic acid, alpha tocopherol, or gamma tocopherol. Any appropriate antioxidant can be used depending on the activities and properties as determined by a person of skill in the art. The antioxidant can be used dry or wet.

In some embodiments, nitric oxide can be delivered to the patient for a period between 15 minutes and 48 hours or between 12 hours and 36 hours.

In some embodiments, nitric oxide can be delivered to the patient for a period of at least 15 minutes, at least 30 minutes, at least 45 minutes, at least 1 hour, at least 2 hours, at least 4 hours, at least 6 hours, at least 8 hours, at least 12 hours, at least 24 hours, at least 36 hours or at least 48 hours.

In some embodiments, nitric oxide can be delivered to the patient for a period of at most 15 minutes, at most 30 minutes, at most 45 minutes, at most 1 hour, at most 2 hours, at most 4 hours, at most 6 hours, at most 8 hours, at most 12 hours, at most 24 hours, at most 36 hours or at most 48 hours.

In some embodiments, nitric oxide can be delivered to a patient continuously. In other embodiments, nitric oxide can be delivered to a patient intermittently. Intermittently can mean that the nitric oxide is delivered in pulses or in intervals having higher and lower concentrations of nitric oxide, for example, on-off cycles, or pulsed nitric oxide delivery.

In some embodiments, a method can further include releasing nitrogen dioxide from a nitrogen dioxide source. A nitrogen dioxide source can include a nitrogen dioxide releasing compound, such as dinitrogen tetroxide (e.g. liquid dinitrogen tetroxide). A nitrogen dioxide source can include a gas bottle (e.g. nitrogen dioxide gas bottle). A gas bottle can be a pressurized gas bottle, gas tank or pressurized gas tank.

In some embodiments, a nitrogen dioxide source can be coupled to a cartridge. A nitrogen dioxide source can be coupled to a cartridge either directly or indirect, for example, by tubing.

In some embodiments, a cartridge can include a plurality of cartridges. A plurality can include 1, 2, 3, 4, 5 or more cartridges.

Other features will become apparent from the following detailed description, taken in conjunction with the accompanying drawings, which illustrate by way of example, the features of the various embodiments.

## BRIEF DESCRIPTION OF THE FIGURES

FIG. 1 is a schematic of a cartridge.
FIG. 2 is a schematic of a delivery system including a cartridge.
FIG. 3 is a diagram of a NO delivery system.
FIG. 4 is a diagram illustrating the N2O4 reservoir and critical flow restrictor.
FIG. 5 is a diagram illustrating a standard NO generation cartridge.
FIG. 6 is a diagram illustrating a tube with multiple concentric hollow ribs.
FIG. 7 is a diagram illustrating an expanded view of a tube with multiple concentric hollow ribs.
FIG. 8 is a diagram illustrating a rib.
FIG. 9 includes a cut-away of a reservoir assembly and a perspective illustration of a reservoir assembly.
FIG. 10 is a schematic of a reservoir assembly.
FIG. 11 includes cut-away schematics of a reservoir assembly.
FIG. 12 is a picture of a pressurized metal tube.
FIG. 13 is a schematic of a cap of a cartridge.
FIG. 14 is a schematic of a system for delivering nitric oxide.
FIG. 15 is a schematic of a system for delivering nitric oxide.
FIG. 16 is a schematic of a circuit board.
FIG. 17 is a schematic of a system for delivering nitric oxide including a disposable module.
FIG. 18 includes perspective drawings of a system, a disposable module and a base unit.
FIG. 19 is a picture of a system in use.
FIG. 20 is a graph illustrating temperature versus NO output for a 25 micron diameter ribbed tube packed with ascorbic acid/silica gel powder.
FIG. 21 is a graph illustrating air flow rate versus NO output for a 50 micron diameter ribbed tube packed with ascorbic acid/silica gel powder.

FIG. 22 is a graph illustrating NO and $NO_2$ output for a ribbed flexible tube. The graph further illustrates relative humidity, temperature at the outlet, ambient temperature and $NO_2/NO_x$ ratios.

FIG. 23 is a graph of performance data.

FIG. 24 is a graph of ppm NO, $NO_2$ and NO + $NO_2$ versus time.

FIG. 25 is a graph of stability of output versus time.

FIG. 26 is a graph of NO and $NO_2$ output over a period of time.

## DETAILED DESCRIPTION

When delivering nitric oxide (NO) for therapeutic use to a mammal, it can be important to avoid delivery of nitrogen dioxide ($NO_2$) to the mammal. Nitrogen dioxide ($NO_2$) can be formed by the oxidation of nitric oxide (NO) with oxygen ($O_2$). The rate of formation of nitrogen dioxide ($NO_2$) can be proportional to the oxygen ($O_2$) concentration multiplied by the square of the nitric oxide (NO) concentration. A NO delivery system can convert nitrogen dioxide ($NO_2$) to nitric oxide (NO).

Generally, nitric oxide (NO) is inhaled or otherwise delivered to the individual's lungs. Providing a therapeutic dose of NO would treat a patient suffering from a disorder or physiological condition that can be mediated by inhalation of NO or supplement or minimize the need for traditional treatments in such disorders or physiological conditions. For example, nitric oxide has been used to help treat and diagnose cardiac arrest, as described in U.S. Patent Application No. 12/508,959, which is incorporated by reference in its entirety.

Despite the promise of inhaled nitric oxide in treating patients, patients often require delivery of inhaled NO quickly and in settings in which the currently approved devices are inconvenient, such as a home or an ambulance. The currently approved devices and methods for delivering inhaled NO gas are inconvenient because they can require complex and heavy equipment. NO gas is stored in heavy gas bottles with nitrogen and no traces of oxygen. The NO gas is mixed with air or oxygen with specialized injectors and complex ventilators, and the mixing process is monitored with equipment having sensitive microprocessors and electronics. All this equipment is required in order to ensure that NO is not oxidized into nitrogen dioxide ($NO_2$) during the mixing process since $NO_2$ is highly toxic. However, this equipment is not conducive to use in a non-medical facility setting (e.g., remote locations, at home, while shopping, in a vehicle or at work) since the size, cost, complexity, and safety issues restrict the operation of this equipment to highly-trained professionals in a medical facility.

In addition to the location of a patient, a patient's mobility may be limited by the currently approved devices since the treatment requires bulky and/or heavy equipment. Accordingly, a light, portable, ambulatory device for delivering NO with air has the potential to be transported to the patient, for example, in an ambulance. The device may be powered by a small, battery-driven pump or by patient inhalation (similar to smoking a cigar). Additionally, a treatment providing NO (e.g., converting $N_2O_4$ into NO) may be more cost effective than oxygen therapy.

Devices that can be useful for treating patients are described herein. Advantages of these devices are that the devices can be they require little or no electronics, no monitors and/or no ventilators. Accordingly, these devices can be used on-site to quickly and efficiently deliver NO to patients.

The nitric oxide delivery devices and systems described herein all deliver ultra-pure nitric oxide, with virtually zero of $NO_2$ delivered to the patient. The $NO_2$ levels have been determined to be less than 0.09 ppm using CAPS. The $NO_2$ levels can go down further with better prototypes, with expected levels to be down to 0.05 ppm and below. These levels are lower than the $NO_2$ level in the ambient air. With conventional delivery systems, a four-fold increase in the level of toxic $NO_2$ results from a change in the nitric oxide level going from 10 to 20 ppm and a 16-fold increase in the toxic $NO_2$ level from a change in the nitric oxide level going from 10 to 40 ppm. Because $NO_2$ is known to cause some of the very problems that NO treats, increasing the NO dose has been shown again and again in the literature to be counterproductive.

For example, the harmful effects of $NO_2$ in man, even for short exposures are:

TABLE 1

| DOSE | EXPOSURE | OUTCOME |
|---|---|---|
| 0.1 ppm | 60 min | 13/16 asthmatics increase in reaction to bronchoconstrictor |
| 0.7 - 2.0 ppm | 10 min | Increase in inspiratory and expiratory flow resistance |
| 1.6 - 2.0 ppm | 15 min | Significant increase in total airway resistance |
| 1.6 - 5.0 ppm | 15 min | Increase in airway resistance in patients with chronic bronchitis |

The reported effect on animals exposed to less than 1 ppm is shown below:

TABLE 2

| ANIMAL | DURATION | OUTCOME(S) |
|---|---|---|
| Rat | 1 day | Peroxidation of lung lipids<br>Increased glutathione peroxidase activity<br>Mast cell degradation<br>Reduction of alveolar cell mitochondria increased in lung |
| Mouse | 1 day | Increased ascorbic acid levels in the liver |
| Guinea Pig | 7 days | Increased protein content of lung lavage fluid |
| Rabbit | 24 days | Structural changes in lung collagen fibers |
| Mouse | 30 days | Spleen:<br>    Reduced antibody production<br>Lung:<br>    Edema and reduction in cilia of alveolar epithelial cells<br>    Desquamation<br>    Bronchial adenomatour proliferation<br>    Focal emphysema |
| Rat | 90 days | Lung:<br>    Bronchitis and peri-bronchitis<br>Central Nervous System:<br>    Changes in conditioned reflexes |
| Mouse | 90 days | Depression in serum neutralizing antibody titres |

The devices described can use different hardware to deliver nitric oxide safely to a patient. For example, the hardware could include a permeation tube, for example, as described in U.S. Patent No. 7,560,076 and U.S. Patent Publication No. 2009/0285731 A1, each of which is incorporated by reference in its entirety. The hardware could include a diffusion tube, for example, as described in U.S. Patent Publication No. 2010/0104667 A1 and U.S. Patent Publication No. 2010/0043788 A1, each of which is incorporated by reference in its entirety. For most cases both designs can work, although a permeation tube can take 12 to 36 hours to stabilize as compared to the 1 to 5 minutes for a diffusion tube with a controlled leak.

Nitric oxide generation, uses and additional hardware configurations are described in detail, for example, in U.S. Patent Publication No. 2007/0089739, U.S. Patent No.8,066,904, U.S. Patent Publication No. 2006/0172018, U.S. Patent Publication No. 2009/0285731, U.S. Patent No. 8,057,742, U.S. Patent No. 7,947,227, U.S. Patent No. 7,914,743, U.S. Patent Publication No. 2010/0043787, U.S. Patent Publication No. 2010/0043789, U.S. Patent Publication No. 2010/0043788, U.S. Patent Publication No. 2010/0030091, U.S. Patent Publication No. 2010/0089392, U.S. Patent Publication No. 2010/0104667, U.S. Patent Publication No. 2011/0220103, U.S. Patent Publication No. 2011/0240019, U.S. Patent Publication No. 2011/02362335 or U.S. Patent Publication No. 2011/00259325, each of which is incorporated by reference in its entirety.

A compact, tiny source, low weight, safe, controlled source of NO can be utilized with a specialized section for each disease, or in the case of the Ventilator platform, a group of diseases. For example, a flexible bag operable to inflate and deflate as the mammal breathes may be included, and a cartridge may be positioned between the flexible bag and a point at which the flow having the nitric oxide is delivered to the mammal.

In a preferred embodiment, the nitrogen dioxide/nitric oxide source of any of the devices described below can be heated to a constant temperature in the range of about 35 to 60 °C, for example at about 35 °C, at about 37 °C, at about 40 °C, at about 42 °C, at about 45 °C, at about 50 °C, at about 55 °C or at about 60 °C. This can be done for exquisite control of the process. Body heat can be used, for example, by having the user place the source close to the torso so as to get to the core body temperature of about 37 °C. The location can include a location near the arm pit, between the legs, under the breasts, etc. For people in remote or isolated settings, such as hikers or soldiers, this may be preferable because it can save on energy. This, in turn, can reduce the number of batteries or other energy supply devices that need to be carried.

CARTRIDGE:

One delivery device that could be utilized for quick and simple delivery of nitric oxide to a patient is a nitrogen dioxide bottle coupled to one or more cartridges. A cartridge can also be referred to as cartridge, a NO generation cartridge, a cylinder or a ribbed tube. Cartridges have been described in detail, for example, in U.S. Patent Nos. 7,560,076, 7,618,594 and 8,057,742, each of which is incorporated by reference in its entirety.

A cartridge can employ a surface-active material and a reducing agent. The surface-active material can be coated with the reducing agent. For example, a surface-active material can be coated with an antioxidant as a simple and effective mechanism for making the conversion. The antioxidant can be in an aqueous solution or may be deposited on the surface-active material as an aqueous solution and dried.

$NO_2$ can be converted to NO by passing the dilute gaseous $NO_2$ over a reducing agent, preferably over a surface-active material coated with a reducing agent. When the reducing agent is in the form of aqueous ascorbic acid (that is, vitamin C), the reaction can be quantitative at ambient temperatures. The use of a cartridge should be contrasted with other techniques for converting $NO_2$ to NO. Two such techniques are to heat a gas flow containing $NO_2$ to over 650 degrees Celsius over stainless steel, or 450 degrees Celsius over Molybdenum. Both of these techniques can be used in air pollution instruments that convert $NO_2$ in air to NO, and then measure the NO concentration by chemiluminescence. Another method that has been described is to use silver as a catalyst at temperatures of 160 degrees Celsius to over 300 degrees Celsius. These techniques, while potentially effective, are not reasonable for use in a patient setting, where a cartridge can be used.

A surface-active material can be a material that has a large surface area. Preferably a surface-active material is also capable of absorbing moisture. One example of a surface-active material is silica gel. Another example of a surface-active material that could be used is cotton. The surface-active material may be or may include a substrate capable of retaining water.

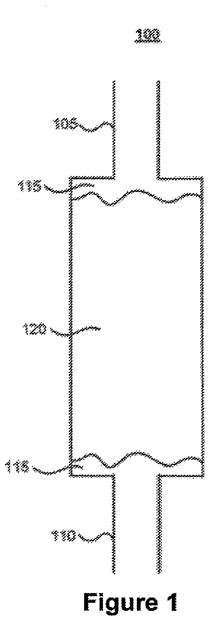

FIG. 1 illustrates a cartridge 100 for generating NO by converting $NO_2$ to NO. The cartridge 100, which may be referred to as a NO generation cartridge, a cartridge, or a cylinder can include a surface-active material 120 and a reducing agent. In addition, a cartridge can include an inlet 105 and an outlet 110. Screen and glass wool 115 can be located at both the inlet 105 and the outlet 110, and the remainder of the cartridge 100 can be filled with a surface-active material 120. The surface-active material can be coated with a reducing agent. For example, the surface-active material can be soaked with a saturated solution of antioxidant in water to coat the surface-active material. The screen and glass wool 115 can also include a reducing agent. Preferably, the screen and glass wool 115 can be soaked with the saturated solution of antioxidant in water before being inserted into the cartridge 100.

In a general process for converting $NO_2$ to NO, a flow having $NO_2$ is received through the inlet 105 and the air flow is fluidly communicated to the outlet 110 through the surface-active material 120 and in contact with the reducing agent. In some embodiments, the surface-active material can remain moist, and if the antioxidant has not been used up in the conversion, the general process can be effective at converting $NO_2$ to NO at ambient temperature.

The inlet 105 may receive the flow having $NO_2$ from a $NO_2$ source that fluidly communicates the flow into a cartridge 100. For example, the inlet 105 may receive the flow having $NO_2$, for example, from a pressurized bottle of $NO_2$, which also may be referred to as a tank of $NO_2$, a gas bottle or a gas bottle of $NO_2$. The flow can include $NO_2$ alone or $NO_2$ in nitrogen ($N_2$), air, oxygen ($O_2$), or an inert gas.

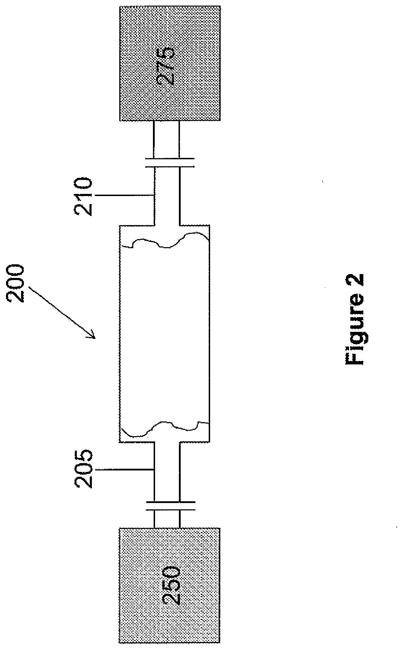

As shown in FIG. 2, the cartridge 200 can be coupled to a $NO_2$ source 250, directly or indirectly. The cartridge 200 can be coupled to a $NO_2$ source 250 via the inlet 205. The cartridge 200 can further be coupled to a patient interface 275, for example, via the outlet 210. A patient interface 275 can include a mouth piece, nasal cannula, face mask, or fully-sealed face mask. In some embodiments, more than one cartridge 200 can be included between a NO2 source 250 and a patient interface 275.

A cartridge can be coupled to a gas bottle as described in U.S. Patent Application Nos. 13/094,541, 29/360,522 and 29/360,525, each of which is incorporated by reference in its entirety.

The conversion of $NO_2$ to NO can occur over a wide concentration range. Experiments have been carried out at concentrations of $NO_2$ in air of from about 2 ppm $NO_2$ to 100 ppm $NO_2$, and even to over 1000 ppm $NO_2$. In one example, a cartridge that was approximately 6 inches long and had a diameter of 1.5-inches was packed with silica gel that had first been soaked in a saturated aqueous solution of ascorbic acid. The moist silica gel was prepared using ascorbic acid (i.e., vitamin C) designated as A.C.S reagent grade 99.1% pure from Aldrich Chemical Company and silica gel from Fischer Scientific International, Inc., designated as S8 32-1, 40 of Grade of 35 to 70 sized mesh. Other sizes of silica gel also are effective. For example, silica gel having an eighth-inch diameter also would work.

The silica gel was moistened with a saturated solution of ascorbic acid that had been prepared by mixing 5-35% by weight ascorbic acid in water, stirring, and straining the water/ascorbic acid mixture through the silica gel, followed by draining. It has been found that the conversion of $NO_2$ to NO proceeds well when the silica gel coated with ascorbic acid is moist. The conversion of $NO_2$ to NO does not proceed well in an aqueous solution of ascorbic acid alone.

The cartridge filled with the wet silica gel/ascorbic acid was able to convert 1000 ppm of $NO_2$ in air to NO at a flow rate of 150 ml per minute, quantitatively, non-stop for over 12 days. A wide variety of flow rates and $NO_2$ concentrations have been successfully tested, ranging from only a few ml per minute to flow rates of up to 5,000 ml per minute. The reaction also proceeds using other common antioxidants, such as variants of vitamin E (e.g., alpha tocopherol and gamma tocopherol). Accordingly, a reducing agent in a cartridge can be any number of known antioxidants, including ascorbic acid, alpha tocopherol and gamma tocopherol.

A cartridge can include a plurality of cartridges. A plurality can be 2, 3, 4, 5 or more cartridges.

A cartridge may include activated alumina. A cartridge may be configured fluidly communicate a flow through activated alumina to trap the gaseous nitrogen dioxide at ambient temperature.

The reducing agent/surface-active material cartridge may be used for inhalation therapy quickly with patients in an ambulatory or home setting. The cartridge may be used as a $NO_2$ scrubber for NO inhalation therapy that delivers NO from a pressurized bottle source. The cartridge may be used to remove any $NO_2$ that chemically forms during inhalation therapy. This cartridge may be used to help ensure that no harmful levels of $NO_2$ are inadvertently inhaled by the patient.

AMBULATORY DEVICES:

Ambulatory devices for use in the treatment methods can be self-contained, portable systems that do not require heavy gas bottles, gas pressure and flow regulators, sophisticated electronics, or monitoring equipment. Additionally, the delivery devices are easy to use and do not require any specialized training. The delivery devices can be also lightweight, compact, and portable. Moreover, the delivery devices can allow an individual to self-administer a NO treatment, if necessary. Ambulatory devices have been described in detail, for example, in U.S. Patent Publication No. 2011/0220103 A1, which is incorporated by reference in its entirety.

According to one embodiment, the NO delivery device can be the size of a coke can for one-time use or short-term treatments lasting from 24 to 200 hours. Alternatively, the treatments can last from 5 to 20 minutes in a catheterization laboratory, to 6 hours during the day, to 24 hours per day to weeks at a time. In another embodiment, the NO delivery device is the size of a cigar or a conventional inhaler. Alternatively, the NO delivery device is a larger device, yet portable device that can deliver NO for longer periods of time. In one embodiment, the NO delivery device can deliver NO for 4 days at 80 ppm NO and a flow rate of 1L/min from a source of only 1 gram of liquid $N_2O_4$ or less than 0.7 mL of $N_2O_4$. In another embodiment, the NO delivery device can deliver NO for several days from a source of only 0.5 gram of liquid $N_2O_4$.

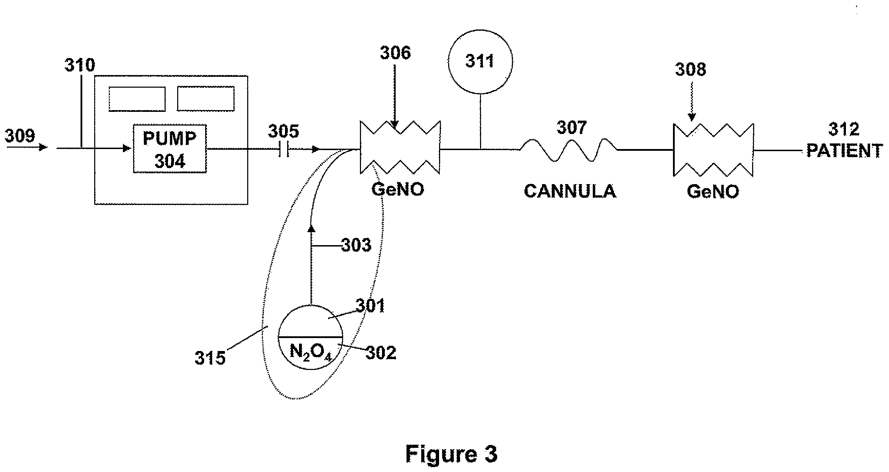

As shown in FIG. 3, the NO delivery system can include a reservoir 301. Generally, the reservoir 301 can supply NO lasting a few minutes to one or more days of continuous use, depending upon the method of storing the NO. In one embodiment, the reservoir 301 can store a therapeutic amount of $NO_2$ that can be converted into NO. The therapeutic amount of NO can be diluted to the necessary concentration while it is still $NO_2$, before the $NO_2$ is converted into NO. The NO can be diluted in air, oxygen, nitrogen or an inert gas. In another embodiment for long-term use for many days, the NO can be stored as liquid dinitrogen tetraoxide ($N_2O_4$) that is vaporizable into $NO_2$, typically, which in turn, can be converted into NO. In various embodiments, the reservoir 301 can be sized to hold a few milligrams to tens of grams of liquid $N_2O_4$. For short-term treatments, the reservoir 301 can be sized to contain a few milligrams of $N_2O_4$. For example, the reservoir 301 may be sized to hold approximately 7 mg of $N_2O_4$ (1), which would provide 20 ppm of NO for ten minutes. For long-term applications, the reservoir 301 may be sized to contain 10 or more g of $N_2O_4$ for long-term use such as several weeks. For example, a reservoir containing approximately 0.3 g of $N_2O_4$ may provide 20 ppm of NO at 20 L/min. for 24 hours, and a reservoir containing 10 g of $N_2O_4$ would provide a continuous supply of NO for approximately 30 days. In other examples, the reservoir 301 can be sized to hold less then 1 ml, 2 ml, 3 ml, 4 ml, 5 ml or 10 ml of liquid $N_2O_4$.

In one embodiment, the reservoir 301 can contain 1 g (about 0.7 ml) of $N_2O_4$ (302). The reservoir 301 can be attached to a tiny orifice or tube with a very narrow bore, 303. The reservoir 301 and the tube 303 can be covered by insulation 315. Since $N_2O_4$ boils at 21°C, the pressure inside the reservoir can be approximately 15 psi at 31°C, 30 psi at 41°C and 60 psi at 51°C for example. Instead of a gas regulator to control the pressure of the gas within a device, the temperature can be controlled such that the pressure inside the device is controlled precisely. As the gas vaporizes, one molecule of $N_2O_4$ can form two molecules of $NO_2$. Using the known physical gas properties of $NO_2$, a critical orifice hole of about 3 to 4 microns would leak out $NO_2$ at about 0.16 ml per minute. If this 0.16ml of $NO_2$ were diluted into a gas stream of 2 liters per minute, the resulting concentration would be 80 ppm (parts per million). The same result can be achieved by using for example, a quartz tube 303 with a 25 micron diameter bore size and about 20 inches long.

The pressure inside the reservoir 301 can be controlled very precisely by controlling the temperature. The flow rate Q out of the reservoir is proportional to the differential pressure, the fourth power of the diameter of the tube, and inversely proportional to the length of the tube. This equation was tested for this application:

$$Q = \Pi\Delta P D^4$$

$$128\mu L$$

In one embodiment, a small ON/OFF valve can be inserted between the reservoir and the fine tube. The valve can act as a variable sized hole. In another embodiment, the quartz tube can be sealed off with a hot flame and have no valve; resulting in an extremely simple device with just a reservoir which is heated to a known temperature and a fine tube. The device can be activated by heating the reservoir and cutting the tube to the desired length.

In another embodiment, the NO delivery system can include a gas pump 304 that blows about 0.5 to 2 L/min of gas through a tube 305. In other embodiments, the gas pump can operate at about 4 to 20 L/min. The heated $N_2O_4$ source can leak $NO_2$ slowly into a stream to form a concentration of about 80 ppm of $NO_2$ in a gas. This can then be passed through a cartridge 306 containing a surface activated material and a reducing agent. If the cartridge is not ribbed and has smooth walls, then the tube may need to be in the vertical position so as to prevent a path whereby the gas could bypass the surface activated material and the reducing agent, to avoid settling of the fine powder.

A second back up cartridge 108 may be located just before the cannula 307. There are three reasons for doing so: First, the second cartridge can convert any $NO_2$ that is formed in the interconnecting tubing back into NO. Second, the second cartridge can provide a doubly redundant $NO_2$ to NO reactor, in case of failure of the first tube, 306. Third, the second cartridge can guarantee the absence of $NO_2$ and therefore can replace the need for having a $NO_2$ monitor for safety purposes. The safety can be further enhanced when the two tubes are made from different batches of surface activated material and reducing agent. A surface activated material can include silica. A reducing agent can be an antioxidant. Examples of an antioxidant can be ascorbic acid, alpha tocopherol, or gamma tocopherol.

FIG. 3 illustrates the gas (e.g. air) intake (arrow 309) and gas intake connection 310 to the gas pump 304. The pressurized gas then leaves the pump. For ambulatory use, this gas flow can be in the range of 0.1 to 5 L/min. In one embodiment, the pump is a battery-driven pump. The gas can also be supplied by a compressor. The gas can also be supplied from a wall outlet, such as in a hospital. Oxygen can be used to replace the gas, provided that the internal components of the system are suitable for use with pure oxygen. The liquid $N_2O_4$ contained in the reservoir 301 can be connected to a cartridge 306 that contains a surface-activated material containing an aqueous solution of an antioxidant, by means of a fine fused capillary tube 303. The tube can be a silica tube, a fused silica tube or a quartz tube. The tube can have a bore size of about 50 microns or less, 25 microns or less, for example, 15 microns, 10 microns or 5 microns. The tube can have a bore size of 10 microns or less. The size of the tube can be chosen based on the concentration that is needed and the flow volume. In one embodiment, to deliver 80 ppm at 20 L, a bore size of 80 microns or more may be required. The tube can be of the type that is used for gas chromatography. The tube may have no interior coating and may be coated on the outside with a polyamide protective layer to prevent the tube from breaking. The tube can be 30 inches long or as little as 0.25 inches so long as the pressure drop across the tube is calculated to provide the correct amount of flux of $NO_2$ to provide the therapeutic dose. Tubing lengths of between 0.1 to 50 inches have been used.

When heated, the liquid $N_2O_4$ can vaporize to $NO_2$ since the boiling point of $N_2O_4$ is about 21°C. The vapour can pressurize the reservoir and a small amount of the $NO_2$ gas can be vaporized through the tube 303 into the first cartridge 306. In, or just before, the first cartridge 306, the $NO_2$ is first mixed with a gas and then converted to NO. The cartridge may also be referred to as a conversion cartridge or converter. Such NO generation cartridges are described above and in U.S. Application Serial No. 12/541,144, which is incorporated by reference in its entirety. The first cartridge 306 includes an inlet and an outlet. In one embodiment, the cartridge can be filled with a surface-active material and a reducing agent. For example, the surface-active material can be soaked with a solution of antioxidant in water to coat the surface-active material. The antioxidant may sometimes be referred to as pixie dust. The antioxidant can be ascorbic acid, alpha tocopherol, or gamma tocopherol or almost any suitable reducing agent. The surface-active material can be silica gel or any material with a large surface area that can be compatible with the reducing agent.

The inlet of the cartridge may receive the flow having $NO_2$. The inlet can also receive a flow with $NO_2$ in nitrogen ($N_2$), air, or oxygen ($O_2$). The conversion occurs over a wide concentration range.

NO gas can then exit from the first cartridge 306. In one embodiment, NO can exit from the first cartridge 306 into a NO sensor 311. The NO sensor can be directly coupled to a nasal cannula tubing 307. The NO sensor can be an optional safety device used to assure that NO gas is flowing. The NO sensor can be a separate NO monitor, or the sensor and the electronics can be mounted in the gas flow path itself. The reason for mounting in the flow path is that there is no need for a separate sample line, and also that the response time of the detector is reduced from multiple seconds to milliseconds.

In a further embodiment, the nasal cannula tubing 307 can be connected to a second cartridge 308 that contains a surface-active material that is soaked with a solution of antioxidant in water to coat the surface-active material. The function of the second cartridge 308 can be the same as the first cartridge 306 and serves as a back-up in case the first cartridge fails to convert $NO_2$ to NO. The mixture can then flow directly to a patient interface 312. The patient interface can be a mouth piece, nasal cannula, face mask, or fully-sealed face mask. The $NO_2$ concentration in the gas stream to the patient is always zero, even if the gas flow to the cannula is delayed, since the second cartridge will convert any $NO_2$ present in the gas lines to NO.

[0084] It is contemplated that one or more of the components of the system illustrated in FIG. 1 may not be directly connected together. FIG. 3 illustrates that the pump 304 and power module is separate from the $N_2O_4$ reservoir 301 and the first and second cartridges 306 and 308. The power module can be purchased and assembled separately and can have its own battery charger built in or use one way or rechargeable batteries. The pump may be powered from a electrical outlet such as in a home, can be battery operated, solar powered, or crank powered. The $N_2O_4$ reservoir 301 and the first and second cartridges 306 and 308 can be a disposable module. The disposable module can be purchased separately at a pharmacy for example, as a prescription drug. The disposable module can be designed to last for 6 hours, 24 hours, 2 days, 4 days, 7 days, 2 weeks, a month or longer. In one embodiment, with twice the amount of material for both $N_2O_4$ and ascorbic/silica gel combination in the cartridges, the lifetime of the disposable modules can be increased by two-fold.

The system illustrated in FIG. 3 can optionally include a $NO_2$ monitor. The $NO_2$ sensor can be a separate $NO_2$ monitor, or the sensor and the electronics can be mounted in the gas flow path itself. One reason for mounting in the flow path can be that there is no need for a separate sample line, and also that the response time of the detector is reduced from multiple seconds to milliseconds. For $NO_2$ it can be especially important that the sample lines be kept as short as possible, since $NO_2$ "sticks" to the tubing walls and as a result the time constant of the system can be very long, for example minutes to hours. Having an inline sensor can eliminate this problem.

The NO and NO sensor can be calibrated periodically and also checked periodically to ensure that they are fully functional and have not failed and/or are still in calibration. Calibration and checking can be tedious and time consuming and there is no insurance that the calibration had failed immediately after the previous calibration. For this reason it is desirable to auto calibrate the sensors. One method which has been successful is to supply a very short time spike of NO and/or $NO_2$, such that the duration of the spike is only a few milliseconds. This is enough time to have the computer recognize the time frequency and magnitude of the spike and use the result as a calibration check.

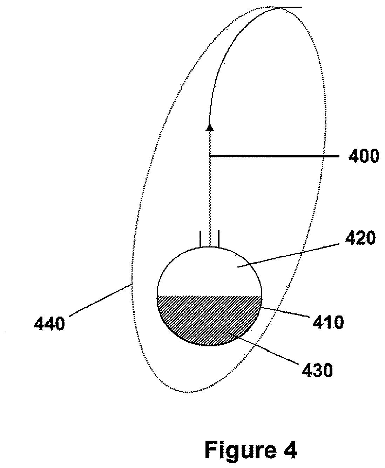

**$N_2O_4$ reservoir and critical flow restrictor**: FIG. 4 is a diagram illustrating the $N_2O_4$ reservoir 410 and critical flow restrictor tube 400. The reservoir 410 can be spherical or nearly spherical or tubular. The reservoir 410 can be made from a material that is chemically stable against $N_2O_4$. Based on the chemical properties, the reservoir can be manufactured out of fused silica (a high grade of quartz), aluminium or stainless steel. The reservoir can be made from a non-reactive metal such as palladium, silver, platinum, gold, aluminium or stainless steel.

The spherical shape may not only be the strongest physically, but with the exit tube protruding to the center, the spherical shape can allow for operation in any direction with the liquid level never in contact with the tube 400 itself, thereby preventing liquid from being expelled from the system. Other shapes including geometric shapes, tubular shapes, cube shapes can be used as determined by a person of skill in the art.

The reservoir 410 and the capillary tube 400 need to be heated to provide the pressure to drive the $NO_2$ out of the reservoir. In one embodiment, the delivery system illustrated in FIG. 3 and/or 4 can include a heating element for use in cold weather environments (e.g., less than approximately 5°C or those temperatures in which the antioxidant-water combination would freeze and or the $N_2O_4$ would freeze). The heating element can be associated with the reservoir. The heating element may be electrically, chemically, or solar powered. For example, the heating element can be a 20 watt heater which can be an Omega Stainless Steel Sheath Cartridge Heater. The system can also include a thermoelectric cooler so that the system can both be heated and cooled. Such devices are available commercially and provide the ability to rapidly change the temperature. Alternatively, the reservoir or delivery system can be strapped or otherwise held close to an individual's body in order to utilize the individual's body heat to keep the system at operating temperatures (i.e., those temperatures that where $NO_2$ has sufficient vapour pressure and ascorbic acid-water remains a liquid), and to ensure that the dose of NO is adequate.

At 21°C, the pressure in the reservoir 410 can be equal to atmospheric pressure since the $N_2O_4$ (reference 430 in FIG. 4, boils at this temperature). At 30°C the vapor pressure above the liquid would be equal to about 2 atmospheres. This can increase to approximately 4 atmospheres at 40°C and 8 atmospheres at 50°C. Pressures like this can be sufficient to drive the vapor out of the storage vessel 410 and through the 25 micron bore tube 400 and into the gas stream at the cartridge wherein $NO_2$ is converted into NO.

[0091] The pressure has been shown experimentally to approximately double every 10°C, which is expected from theory. Thus, to maintain a constant pressure and therefore a constant driving force, the temperature of the assembly 420 can be controlled. A 1.0°C rise in temperature can cause the pressure to increase by about 10% and therefore the concentration in the air stream to increase by 10%. In order to maintain a constant flow rate to within approximately +- 5%, the temperature at the reservoir needs to be held constant to within 0.25°C.

One limitation on the amount of $N_2O_4$ that the reservoir 410 can contain is related to the consequences in the event of a catastrophic failure where all the liquid $N_2O_4$ suddenly escapes into the room and vaporizes to $NO_2$. If this were to ever happen, then the $NO_2$ level in the room should not exceed 5 ppm, which is the OSHA standard for the workplace. In a standard room defined in FDA Guidance document "Guidance Document for Premarket Notification Submissions for Nitric Oxide Delivery Apparatus, Nitric Oxide Analyzer and Nitrogen Dioxide Analyzer dated 24 January 2000, a room is cited as 3.1 x 6.2 x 4.65 meter room, without air exchange. In order to meet this guideline, the maximum amount of $N_2O_4$ that can be contained in the reservoir would be about 1gram, or 0.7 ml, which would last for about 4 days.

While the safety code was written for high pressure gas bottles where the pressure is typically greater than 2000 psi, it can be much less likely to happen when the internal pressure is only 8 atmospheres, which is equivalent to only 112 psi. Indeed, high pressure gas bottles can be considered empty when the pressure falls below 150 psi. Another approach for exceeding this limit, a storage vessel that can include a reservoir 410 and tube 400 can be surrounded with an alkaline solution 440 that can neutralize the acidic $N_2O_4/NO_2$ in case of a leak. In the event of a catastrophic rupture, the reservoir 410 can be designed to leak into the surrounding alkaline solution, thereby neutralizing the toxic $N_2O_4$. Alkaline solutions can be any solution with a pH higher than 7. Any alkaline solution can be used, including but not limited to calcium oxide (flaked lime), sodium hydroxide, sodium carbonate, potassium hydroxide, ammonium hydroxide, sodium silicate. The same alkaline solution can also be used to neutralize any residual $N_2O_4$ after use or if the system was discarded prematurely. In another example, activated charcoal can be used to absorb $NO_2$ and can be used in packaging.

In another embodiment, the $N_2O_4$ and the reservoir can be heated to about 50°C or higher in order to stabilize the pressure in the storage vessel. A heating element can be used. The heating element may be electrically, chemically, or solar powered. In one embodiment, chemical energy from an exothermic reaction can be used to provide the heat. One compound which could provide this energy is powdered calcium oxide (CaO). When mixed with water it releases energy in the form of heat. This material is also the slaked lime that is used in concrete. It has also been packaged in a format to heat foodstuffs. The added advantage of this material is that it is also alkaline, and the same material can be used to neutralize the $N_2O_4/NO_2$ in the scenario described above.

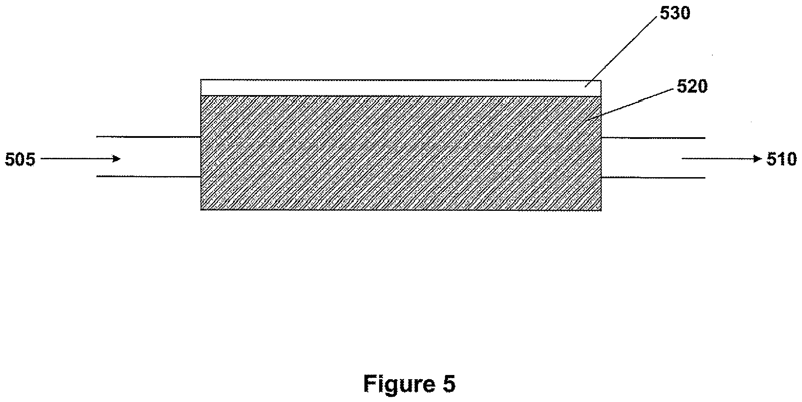

**Packed tube:** In a general process for converting $NO_2$ to NO, an air flow having $NO_2$ can be received by a standard NO generation cartridge through an inlet 505 and the air flow is fluidly communicated to an outlet 510 through the surface-active material 520 coated with the aqueous antioxidant as illustrated in FIG. 5. Typically, when a tube is packed with a powder, the powder can tend to settle, much like a cereal box with corn flakes. Settling can occur due to vibration that is encountered during shipping, as well as during normal use. This can especially be the case when the powder is fragile, like corn flakes, and cannot be well packed or when it is not possible to tightly compact the powder. For example, in packed columns for liquid chromatography, the powder can be packed and used at great pressures; these columns can be usually packed as a slurry to force the powder to be tightly packed. If the powder has an active surface material, such as silica gel, activated charcoal, activated carbon, activated alumina or dessicants such as calcium sulphate (DRIERITE™), to name just a few, and if it is desired to flow gas through the cartridge so that it comes into contact with the active surface, then the powder cannot be packed too tightly or the packed material can fracture, and can allow gas to flow freely without creating too large of a pressure drop. In these cases, the technique that is used commercially today is to pack the powder and try and keep it tightly packed by means of a spring. In addition, the tubes have to be used vertically, so that as the powder settles, there will be no free gas path, 530, which the gas can take to bypass the reactive bed 520, as shown in FIG. 5. If the tubes are not used vertically, then settling of the powder creates a channel 530, across the tube where the gas can flow preferentially. Creation of a channel can negate the effect of the powder and can render the cartridge useless. This problem can be so severe that a packed tube like this can only be used if the cartridge is vertical.

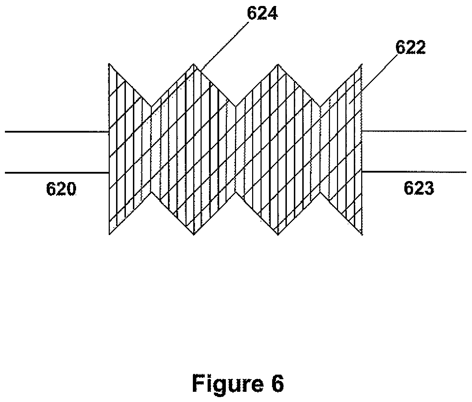

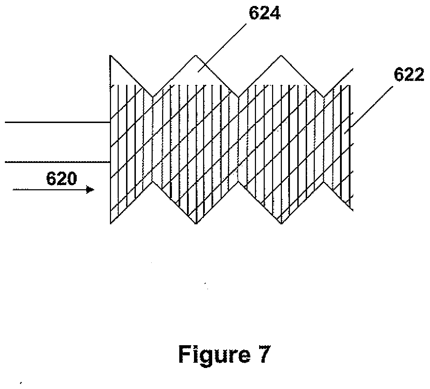

FIG. 6-8 illustrates a tube with multiple concentric hollow ribs that overcomes this problem and allows for a powdered cartridge to be used at any angle, even after it has been exposed to severe vibrations. The tube can be used for all surface-active material including but not limited to silica gel, activated charcoal, or Drierite. The tube can be packed vertically and the powder, 622, is allowed to fill from the bottom to the top, also filling up all the volume enclosed by the ribs. If the tube were then vibrated and placed horizontally, the powder in the ribs could settle, as shown in 624. However, as long as the ribs are deep enough, the gas would not have a preferred channel. Gas flow would find the path to the settled volume more difficult than travelling though the powder bed.

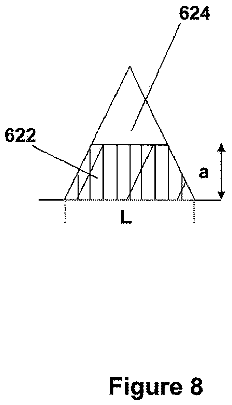

FIG. 8 shows the close up detail of one of the ribs of one embodiment of a cartridge. For simplicity, the ribs are drawn as triangles, although in practice they can have rounded corners and a round top. L is the length at the base of the triangle, and A is the height of the powder above the base. As long as L is always less than 2A, the preferred path for the air would be L, and not A. However, if the decrease in volume was so large that L was greater than 2A, then the air channel in the rib would be the path of least resistance and the air would travel up into the channel, across the channel and down the other side to the next rib.

In one embodiment, the cartridge can be scaled up to be used in a packed bed reactor. At the present time powdered bed reactors are all situated vertically so as to avoid the problem. With the ribbed design, they can be situated at any angle, including horizontally.

Additional ambulatory devices are described in U.S. Patent Application No. 13/094,535, which is incorporated by reference in its entirety.

ULTRA-PURE DELIVERY DEVICE:

As one solution, a system can include a permeation tube or permeation cell to provide the source of $NO_2$. For example, the $NO_2$ source can be liquid dinitrogen tetroxide (N2O4). This approach has been shown to work well. This approach has been described in U.S. Patent Publication No. 2010/0104667, which is incorporated by reference in its entirety. N2O4 can vaporize to produce $NO_2$, and the process can be reversible. Using a permeation tube, air can be allowed to flow around the permeation tube, where it can mix with the $NO_2$ that diffuses through the tube, providing a stable mixture of $NO_2$ in air. The concentration of the $NO_2$ can be controlled by a number of factors including, for example, the temperature of the tube and the volume of the air flow. However, storing a permeation tube can be a problem. For instance, if $NO_2$ is in contact with the permeation tube polymer, the storage should be below -11 °C in order to keep the $NO_2$ frozen, which can prevent loss of $NO_2$. One solution is to build a separate storage chamber for the permeation tube, which can be connected to the storage tube by a simple valve. This device can be stored at room temperature without loss of $NO_2$, and it can easily be activated by connecting the reservoir to the permeation tube. The combined storage vessel and permeation tube can work well, but it can have one major disadvantage. Stabilization of a permeation tube can take a long time when the $NO_2$ is stored in a reservoir and then suddenly opened to the permeation tube. The time to stabilize can be several days. Pre-saturating the permeation tube with $NO_2$ first can speed up the stabilization, but this may not work well with long term storage of months or years.

As another solution, a reservoir assembly can be utilized. Reservoir assemblies have been described in detail in U.S. Patent Publication No. 2011/0259325, which is incorporated by reference in its entirety. A reservoir assembly can include a restrictor and a reservoir.

A reservoir can be any compartment or portion of a compartment suitable for holding $N_2O_4$, $NO_2$ or NO, or other compounds which can generate $N_2O_4$, $NO_2$ or NO. The reservoir can hold a liquid or a solid, but preferably the reservoir can hold liquid $N_2O_4$. The reservoir can be made of any material, which does not react with or adsorb $N_2O_4$, $NO_2$ or NO, or other compounds which can generate $N_2O_4$, $NO_2$ or NO. The material should also be able to tolerate heat within the appropriate range, discussed below, and repeated heating and cooling.

A reservoir can include a nitrogen dioxide source. A nitrogen dioxide source can include $N_2O_4$, $NO_2$, or compounds which can generate $NO_2$. Preferably, the nitrogen dioxide source can contain liquid $N_2O_4$. In the case of liquid $N_2O_4$, the amount of liquid $N_2O_4$ in the reservoir can be less than about 5.0 g, less than about 2.0 g, less than about 1.0 g, less than about 0.50 g, less than 0.25 g or less than 0.10 g; the amount of liquid $N_2O_4$ in the reservoir can be greater than about 0.05 g, greater than about 0.10 g, greater than about 0.20 g, greater than about 0.50 g or greater than about 1.0 g. The amount of liquid $N_2O_4$ in the reservoir can be less than about 5 ml, less than about 2 ml, less than about 1 ml, less than about 0.5 ml, less than about 0.25 ml or less than about 0.10 ml; amount of liquid $N_2O_4$ in the reservoir can be greater than about 0.001 ml, greater than about 0.01 ml, greater than about 0.05, greater than about 0.10 ml, greater than about 0.25ml, greater than about 0.50 ml or greater than about 1.0 ml.

In one exemplary embodiment, liquid $N_2O_4$ can be stored in a small reservoir. For a delivery concentration of 80 parts per million in 1 liter of air per minute, for example, the amount of $N_2O_4$ needed for a 24 hour supply can be approximately 0.24 g, or 0.15 ml. $N_2O_4$ boils at 21 °C, so the device should be heated to above this temperature in order to have a vapor pressure of $NO_2$ that is greater than atmospheric pressure. Further description may be found in U.S. Provisional Application Nos. 61/263,332 and 61/300,425, each of which is herein incorporated by reference in its entirety.

A reservoir can also include nitrogen dioxide vapor or gas in a space over the nitrogen dioxide source.

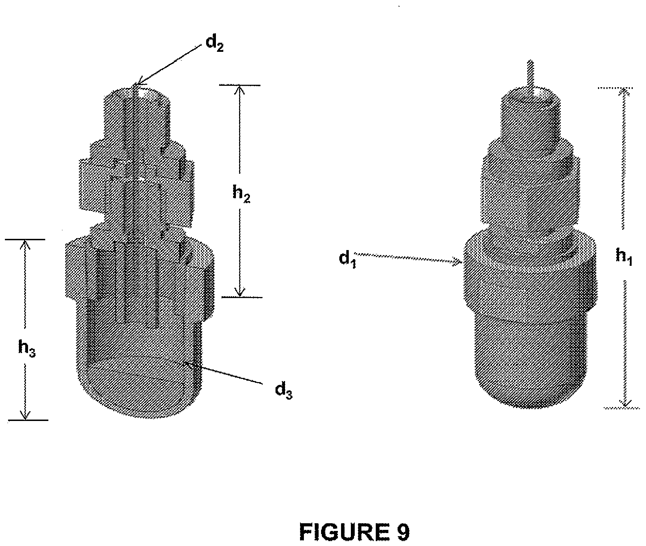

A reservoir can be any size. The size of the reservoir can depend on how the reservoir will be used. It can also be dependent on the amount of the nitrogen dioxide source, the amount of nitrogen dioxide gas required, or the length of the time over which a flow of nitrogen dioxide would be required. A reservoir can be relatively large, for example, greater than 1 foot, greater than 2 feet, greater than 5 feet, or greater than 8 feet in height (h3, FIG. 9). A reservoir can also be relatively small, for example, less than 2 feet, less than 1 foot, less than 6 inches, less than 4 inches, less than 3 inches, less than 2 inches, less than 1 inch, less than 0.5 inch in height (h3, FIG. 9). An assembly can have a size that can accommodate a reservoir and/or additional elements, such as a restrictor. An assembly can be relatively large, for example, greater than 4 inches, greater than 6 inches or greater than 1 foot in internal diameter (d3, FIG. 9). An assembly can be relatively small, for example, less than 4 inches, less than 2 inches, less than 1 inch, less than 0.75 inch or less than 0.5 inch in internal diameter (d3, FIG. 9).

A restrictor can be any device which can limit the flow of $NO_2$ from the reservoir. A restrictor can require that there be enough vapour pressure to force the $NO_2$ vapor out of the reservoir and into the restrictor.

The reservoir can include the restrictor. For example, the restrictor can be an orifice. The restrictor can be coupled to the reservoir. For example, the restrictor can include a tube, most preferably, a capillary tube. The capillary tube can be a quartz capillary tube. The capillary tube can be a narrow bore capillary tube, which can allow for simple, reproducible and accurate use, as well as a cost effective solution. A convenient commercially available restrictor can be a narrow bore quartz tubing that can be used for gas chromatography (GC).

A restrictor can include a first end and a second end. In some embodiments, the first end of the restrictor can be coupled to a reservoir and the second end can be sealed or closed. In some embodiments, the second end, which was previously sealed or closed, can be opened, unsealed or include a broken seal. In some embodiments, a restrictor can further include a length corresponding to the distance between the first end and the second end.

A restrictor can have any dimension, so long as the total pressure drop across the restrictor can be appropriate for the flow of $NO_2$ that is required. In some embodiments, the length of the restrictor can be relatively long, for example, greater than 4 inches, greater than 6 inches, greater than 1 foot, greater than 2 feet, greater than 5 feet, greater than 10 feet or greater than 20 feet long. In some embodiments, a restrictor can be relatively short, for example, at least about 0.1 inch, at least about 0.25 inch or at least about 0.5 inch; the length can be at most about 4 inches, at most about 2 inches, at most about 1 inch, or at most about 0.5 inch. Preferably, the restrictor can have a length of about 0.75 inch. In some embodiments, the internal diameter of the restrictor can be relatively large, for example, greater than about 0.100 microns, greater than about 1 microns, greater than about 5 microns, greater than about 10 microns, greater than about 50 microns or greater than about 100 microns. In some embodiments, the internal diameter of the restrictor can be relatively small, for example, at least about 0.001, at least about 0.005 microns or at least about 0.010; the internal diameter can be at most about 0.100 microns, at most about 0.050 microns, at most about 0.025 microns, or at most about 0.010 microns. Preferably, the restrictor can have a diameter of about 0.010 microns.

The amount of material (e.g. nitrogen dioxide) that is forced out of the reservoir at any temperature can be dependent upon the diameter of the restriction. Thus, the two key design variables can be the temperature of the vessel and the diameter and length of the restriction in the top of the vessel. For example, at about 45 °C a tube of 0.010 microns internal diameter and 0.75 inches long was used to provide 80 ppm of $NO_2$ in an air stream of 11/min.

The restrictor can be made of other materials known to those of skill in the art. The material should not react with or adsorb $N_2O_4$, $NO_2$ or NO, or other compounds which can generate $N_2O_4$, $NO_2$ or NO. The material should also be able to tolerate heat within the appropriate range, discussed below, and repeated heating and cooling.

A restrictor can be sealed. For example, if the restrictor is made of quartz or glass, one end of the restrictor can be heat sealed or melted to close off the opening on that end of the restrictor. The sealed end of the restrictor can be opened by breaking off the end, which can permit a channel in the restrictor to be fully opened. The restrictor can be bevelled or scored to allow for an easier and cleaner break. A restrictor can also be sealed with a metal seal. A metal seal can be melted, punctured, peeled off or otherwise removed to open the sealed end (i.e. break the seal). A restrictor can include a valve, for example, a micromachined valve. Other suitable seals and methods for controlling or preventing flow are known to those of skill in the art. Once the sealed or closed end is opened, nitrogen dioxide can traverse the length of the restrictor and out the previously closed or sealed end.

A reservoir assembly including a reservoir and a capillary can be less than 1 foot, less than 6 inches, less than 5 inches, less than 4 inches, less than 3 inches or less than 2 inches in height (h1, FIG. 9). In an exemplary embodiment, the assembly can be approximately 1.6 inches in height. An assembly can also be less than 1 inch, less than 0.75 inch or less than 0.5 inch in diameter (d1, FIG. 9). In an exemplary embodiment, the assembly can be approximately 0.4 inch (e.g. 0.43 inch) in diameter.

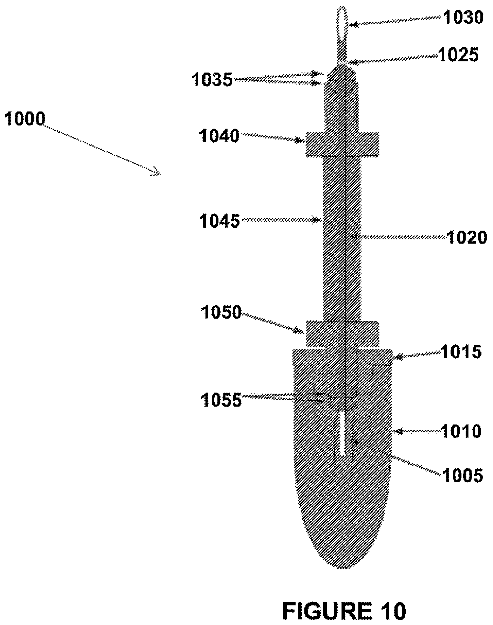

Referring to FIG. 10, in one embodiment of a reservoir assembly, a restrictor can be a capillary 1020, which can be about 1-inch x 10 um internal diameter (TSP010375 Flexible Fused Silica Capillary Tubing Polymicro Technologies). The capillary 1020 can be inserted through a metal (303 S.S.) tube 1045 made up of two GC nuts 1040 and 1050 (1/16" Stainless Steel Nut Valco P/N ZN1-10) connected via their tops to a metal tube 1045. Two graphite ferrules 1055 (Graphite Ferrules P/N 20227 1/16" X 0.4mm Restek) with their flat ends touching can be placed on one end of the capillary 1020, which has the polyamide coating 1005 removed below the ferrules 1055 (e.g., by burning off the polyamide with a flame). The ferrules 1055 can hold the capillary 1020 securely when the nut 1040 is inserted into a separate female end of an adaptor 1015, which can be itself inserted into the metal (303 S.S.) reservoir container 1010. The adaptor 1015 can have a metal sheath 1045 on the reservoir end that can cover and protect the area of the capillary without polyamide.

The end of the capillary 1030 opposite the reservoir adaptor can be flame sealed and scored. The sealed capillary can be tested with a helium flow to assure that the assembly is appropriately sealed and does not leak. The reservoir 1010 can be filled with liquid $NO_2/N_2O_4$ by distillation or other means. The capillary 1020 is attached to the reservoir by means of a 1/8 inch pipe thread and sealed. The reservoir assembly can be heated and checked to assure that there are no $NO_2$ leaks.

The entire liquid reservoir assembly can be heated. Methods for heating the assembly can include: 1) a hot water bath, 2) a heating mantle that straps onto the tubes, insulating the outside of the metal tubing with urethane or another insulator such as paint, and wrapping Kanthal heating wire around the device, and/or 3) using silver paint to paint the heating element onto top of the insulating paint.

The reservoir assembly 1000 can then be attached to the delivery conduit by inserting the sealed end of the capillary 1030 with two ferrules 1035 (Graphite Ferrules P/N 20227 1/16" X 0.4mm Restek) with their flat ends touching and screwing the exposed GC nut 1040 of the reservoir assembly into the delivery conduit.

The sealed end of the capillary 1030 can be inserted into an off-center hole of the internal delivery seal. When ready to use, the internal delivery seal can be rotated to open the reservoir port to the system flow path, which can break the capillary at its scored end 1025, thus opening the reservoir to the system flow path and starting the flow of $NO_2$.

An advantage of having the capillary tube inside the reservoir and protected by the tubing can be that the toxic $N_2O_4$ can only escape through the narrow bore quartz tube. In order for any material to escape the heater has to be turned on to provide the driving force. The tiny liquid reservoir assembly (FIG. 9), which can measure, for example, about 1.6 inch in height and 0.43 inches in diameter, can replace a large pressurized gas cylinder, the gas regulator and the gas control valve. The size can be similar to that of a cap for a ball point pen.

The assembly can be kept the $N_2O_4$ frozen solid at dry ice temperatures. However, while this is suitable for laboratory use, it may be impractical as a safe medical delivery device for use with a patient.

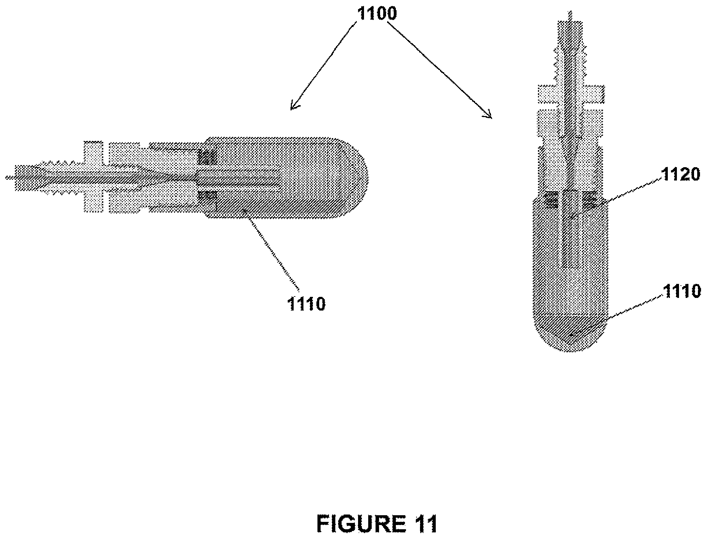

FIG. 11 includes an alternative embodiment. The can include a reduced number of parts, but the overall concept can remain the same. This embodiment can be less expensive to produce. The size and shape of the vessel 400 can be such that the liquid 410 can never enter the restrictor 1120, e.g. capillary tube. In FIG. 11, the vessel 1100 is on its side, and the liquid level 1110 can remain below the level where it could enter the restrictor 1120. Similarly, the vessel 1100 can be inverted and it can still function. The restrictor 1120 can be protected by a wider bore splash guard. A baffle (not shown) can also be placed in front of the restrictor 1120 so as to eliminate the possibility of a minute droplet entering the restrictor 1120.

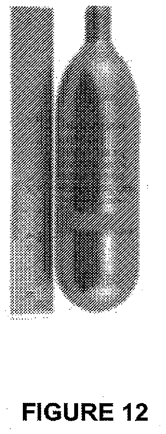

In another embodiment, methods that are used to seal carbon dioxide in metal tubes for a wide variety of commercial and consumer applications can be used (FIG. 12). The liquid $NO_2$ can be sealed inside a steel or aluminum canister, similar to those used for carbon dioxide (see Leland corporation). These devices can have a welded cap made of a thin sheet of steel. The welding can be carried out by resistance heating or other techniques. The advantage of this system can be that the liquid can be sealed inside the container and the containers can be safely shipped. For this application, the volume of the nitrogen dioxide source should be less than 5 ml, less than 2 ml, preferably less than 1 ml. Alternatively, a crimp seal could be used as long as the seal could take the internal pressure of about 100 psi without leaking. The material can be aluminum or stainless steel.

The loading and cap penetration technique can be identical to what is used for carbon dioxide pellet guns and for the multitude of other uses of these tiny high pressure cylinders.

In one aspect, a system for delivering nitric oxide can include a reservoir, a gas supply and a delivery conduit. A system can further include a restrictor. A reservoir and a restrictor have been described above. In some embodiments, a system can include a reservoir and a restrictor, which are part of a reservoir assembly.

A gas supply can be any suitable source of gas, for example air, oxygen or nitrogen. A preferred gas supply is an air supply, for example, an air pump. For the ambulatory platform an air stream can be provided by a small air pump. An air compressor, an external supply of air or oxygen gas from gas bottles can also be used, including oxygen enriched air for a home oxygen generator. The use of air or oxygen, wet or bone dry, may make no difference to performance, as measured by a constant output over time. However, moist air greatly can extend the life of the reducing agent cartridge (e.g. ascorbic acid cartridge) that the $NO_2$ gas will be passed through to generate the drug, nitric oxide. Nevertheless, the platform can be designed for the worst case, which is bone dry air or oxygen.

The system can further include a delivery conduit. A delivery conduit can include a NO sensor, a $NO_2$ sensor, or a temperature sensor. A NO sensor can include a chemiluminescent detector or an electrochemical sensor. A $NO_2$ sensor can include a chemiluminescent detector or an electrochemical sensor. A temperature sensor can include a thermistor or a thermometer. In some instances, the system can include a pressure sensor or a flow sensor. A delivery conduit can also include other medically relevant devices, for example, a filter for eliminating microorganisms prior to inhalation of NO by a patient. It should also be understood that a delivery conduit can include additional hardware, such as tubing and valves, necessary to fluidly communicate gas (e.g. $NO_2$, NO, air, oxygen, nitrogen, etc.) from one element of the system to another.

The delivery conduit can have an inlet, which can be coupled to the gas source. The delivery conduit can also include an outlet, which can be couple to a patient interface. A patient interface can include a mouth piece, nasal cannula, face mask, or fully-sealed face mask.

If the patient required the co-delivery of oxygen, the air feed can be replaced with oxygen, or a dual lumen cannula can flow both the NO in air and oxygen down parallel lumens to the patient, mixing the NO in the air and the oxygen in the nose.

It is also well within the capability of the technology to add an oxygen conserver to the NO output, thereby extending the life time of the disposable component.

The second end of a restrictor can also be coupled to the delivery conduit. The second end of a restrictor can be coupled to the delivery conduit at a location between the inlet and the outlet of the delivery conduit. A restrictor can further include a length corresponding to the distance between the first end and the second end. In some cases, the second end of the restrictor is coupled to the delivery conduit such that the delivery conduit traverses in a direction perpendicular to the length of the restrictor.

As the second end of a restrictor can be closed, the delivery conduit can include a device for opening the second end or breaking the seal on the second end.

A system can further include a cartridge, as previously described. Using the system as an inhaled NO drug delivery device, the $NO_2$ output in air or oxygen can be passed through a cartridge, which strips out one of the O atoms from the $NO_2$ to produce ultra pure NO.

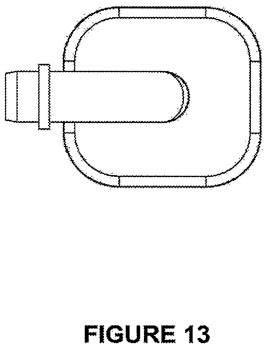

**[0134]** A cartridge can include a cap. The cap for the cartridges can be molded from plastic (FIG. 13).

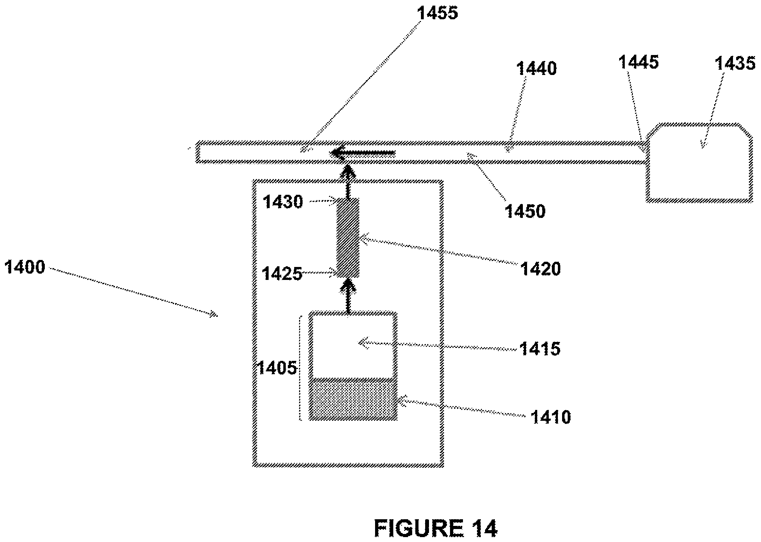

An exemplary embodiment of a system is shown in FIG. 14. Referring to FIG. 14, a system 1400 can include a reservoir 1405. A reservoir 1405 can include a nitrogen dioxide source 1410, for example, liquid $N_2O_4$. Over the nitrogen dioxide source can be nitrogen dioxide vapor 1415. As the vapor pressure of the nitrogen dioxide vapor 1415 is increased, for example by heating the nitrogen dioxide, the nitrogen dioxide 1415 can be forced into a restrictor 1420. The restrictor 1420 can be coupled to the reservoir at a first end 1425. The second end 1430 of the restrictor can be closed or sealed for storage. To use the system, the second end 1430 can be opened or the seal can be broken, which can allow nitrogen dioxide to traverse the length of the restrictor 1420 and out the second end 1430. A gas supply 1435 can provide gas 1450, which can traverse through a delivery conduit 1440. An inlet 1445 of the delivery conduit 1440 can be coupled to the gas supply 1435. The second end of the restrictor 1430 can also be coupled to the delivery conduit 1440. In that way, as gas 1450 from the gas supply 1435 traverses through the delivery conduit 1440 and past the second end of the restrictor 1430, the gas 1450 from the gas supply 1435 and the nitrogen dioxide vapor 1415 from the reservoir will mix, forming a nitrogen dioxide-gas mixture 1455. The nitrogen dioxide-gas mixture can then pass through a number of devices including, but not limited to, sensors, cartridges or filters, as discussed below.

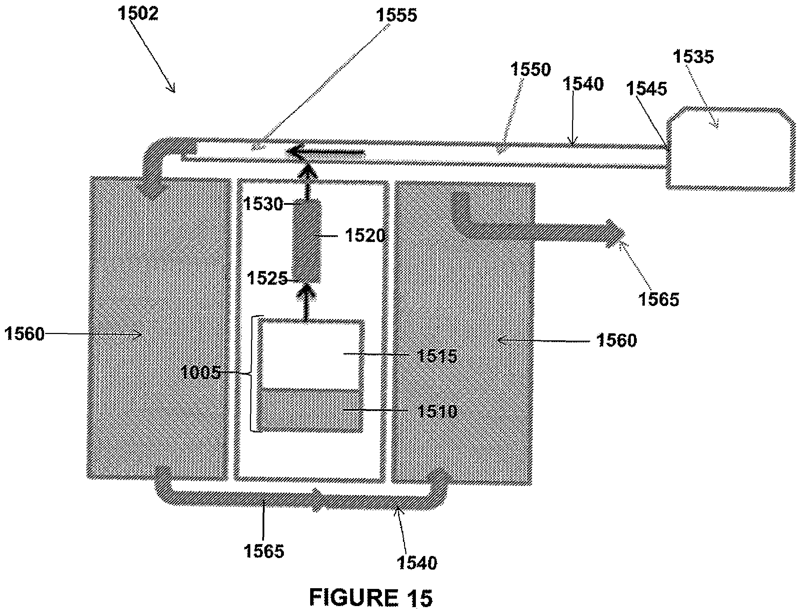

Another exemplary embodiment of a system is shown in FIG. 15. Referring to FIG. 15, a system 1500 can include a reservoir 1505, which can include a nitrogen dioxide source 1510, for example, liquid $N_2O_4$. Over the nitrogen dioxide source can be nitrogen dioxide vapor 1515. As the vapor pressure of the nitrogen dioxide vapor 1515 is increased, for example by heating the nitrogen dioxide, the nitrogen dioxide 1515 can be forced into a restrictor 1520. The restrictor 1520 can be coupled to the reservoir 1505 at a first end of the restrictor 1525. The second end 1530 of the restrictor can be closed or sealed for storage. To use the system, the second end 1530 can be opened or the seal can be broken, which can allow nitrogen dioxide to traverse the length of the restrictor 1520 and out the second end 1530. A gas supply 1535 can provide gas 1550, which can traverse through a delivery conduit 1540. An inlet 1545 of the delivery conduit 1540 can be coupled to the gas supply 1535. The second end of the restrictor 1530 can also be coupled to the delivery conduit 1540. In that way, as gas 1550 from the gas supply 1535 traverses through the delivery conduit 1540 and over the second end of the restrictor 1530, the gas 1550 from the gas supply 1535 and the nitrogen dioxide vapor 1515 from the reservoir will mix, forming a nitrogen dioxide-gas mixture 1555. The nitrogen dioxide-gas mixture 1555 can then pass through a first cartridge 1560 included in the delivery conduit. Prior to or following a cartridge 1560, the nitrogen dioxide-gas mixture 1555 can pass through a number of devices which can be included the delivery conduit including, but not limited to, sensors or filters, as discussed in more detail below. The nitrogen dioxide-gas mixture 1555 can also pass through a second cartridge 1560 prior to exiting the delivery conduit. A patient interface can be coupled to an outlet 1565 of the delivery conduit.

A system can include a heating element. A heating element can be any device that can alter and maintain the temperature of the system, or at least the reservoir and/or the restrictor. The heating element can be a hot water bath, a heating mantle or heating wire. Insulated heating wires can be wrapped directly onto the tube surface. A heated well can also be used. Other suitable examples of a heating element are known to those of skill in the art.

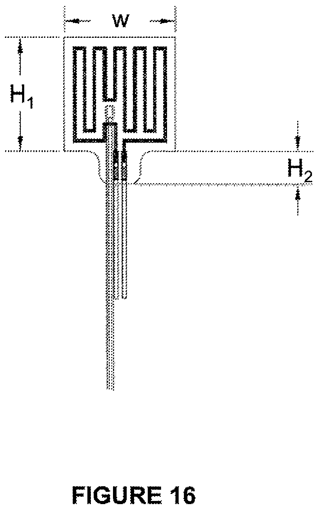

In an exemplary embodiment, the system or a portion of the system, for example the reservoir and/or restrictor, can be heated by means of a simple flexible circuit board with the wires etched onto the surface (FIG. 16). A device including a thermistor can be built into the circuit for measuring and controlling the temperature.

When heating a system or a portion of a system, the lowest temperature that is practical can be about 25 °C. However, it can be difficult to control the temperature precisely when it is close to ambient temperature. For maximum control, the temperature should be set to be above the highest possible ambient temperatures. The upper temperature limit can, in principle, be many hundreds of degrees centigrade. A practical limit can be the engineering balance of (a) having the liquid hot enough to develop the pressure that can force the vapor out of the device, and (b) minimizing the amount of energy that may be needed, especially for battery powered devices, minimizing the amount of thermal insulation that may be needed (a size factor) and the complexity of the storage vessel as far as ensuring that it can withstand the pressures that may be developed inside the vessel. The temperature can be at least about 25 °C, at least about 30 °C, at least about 35 °C, at least about 40 °C, at least about 45 °C or at least about 50 °C; the temperature can be at most about 200 °C, at most about 150 °C, at most about 100 °C, or at most about 75 °C. The optimum temperature range can be about 45 to 75 °C, which can develop enough vapor pressure to force the $NO_2$ vapor through the restrictor.

The reservoir and/or the restrictor can be heated. The reservoir and the restrictor can be heated to substantially the same temperatures, for example less than 10 °C difference, less than 5 °C difference, 2 °C difference or less than 1 °C difference between the temperature of the reservoir and the temperature of the restrictor. This can avoid condensation of $NO_2$. Also, the temperature of the system, more specifically, the reservoir and/or the restrictor, can be controlled to better than about 1 °C, preferably better than about 0.5 °C, in order to maintain a constant output of $NO_2$ vapor. The

higher the temperature of the vessel, the better the temperature control should be. This need can come about because the vapor pressure can approximately double with a 10 degree rise in temperature. Thus, for a fixed restrictor and fixed air flow, the concentration of $NO_2$ in the output can double from approximately 40 ppm at 45 °C to 80 ppm at 55 °C, to 160 ppm at 65 °C to 320 ppm at 75 °C. At 65 °C, a 0.5 °C variation in temperature can cause a change in output that is more than 4 times greater than at 45 °C.

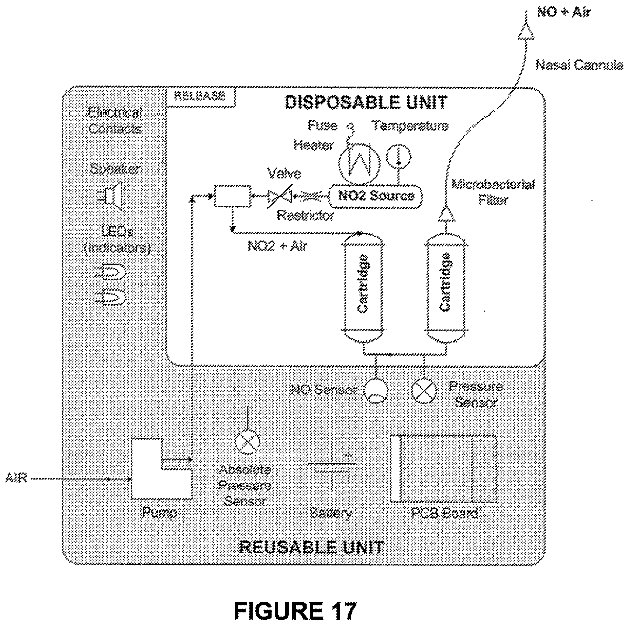

In one embodiment, a portion of the system can be reusable and a portion of the system can be disposable. For example, a reusable base unit can include a gas supply (e.g. air pump). A reusable base unit can also include sensors, power supply (e.g. batteries), alarm systems, lights, indicators, and/or electronics (FIG. 17). A disposable unit can include reservoir, the nitrogen dioxide source (e.g. $N_2O_4$ storage vessel), restrictor and/or at least one cartridge (e.g. two cartridges). The disposable unit can further include filters, a heating element, and/or sensors. One purpose of the design can be to make the disposable system as low cost as possible, while ensuring safety. The liquid $N_2O_4$ source and the at least one cartridge can be contained in a sealed unit that can be produced in large quantities. A typical patient can use one disposable unit per day, which can depend upon the size of the reservoir, the amount of the nitrogen dioxide source, the size of the cartridges, and the dose required.

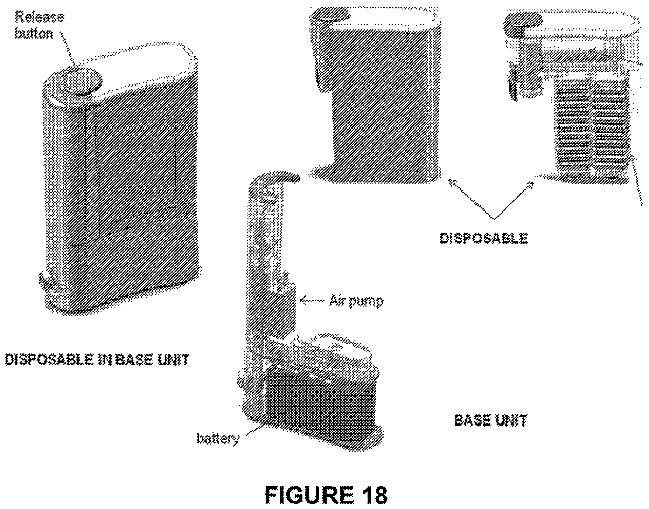

In one embodiment, between the two cartridges, the flow path can pass over an NO sensor (P/N NO-D4 Alphasense, Ltd. United Kingdom), which can verify that the NO levels do not exceed or fall below specified levels. If necessary, the sensor can trigger alarms or shut off the gas supply. One embodiment is shown below in FIG. 18, which shows the base and the disposable, separately and combined.

Some of the safety features of the disposable/reusable system can include the following: 1) an activated charcoal filter on the air intake prior to the valve which breaks off the quartz tip, where the charcoal filter could be large enough to adsorb all of the $NO_2$ in the reservoir; 2) a tip enclosed in a sealed Teflon chamber during shipment, which can only be moved by inserting the disposable unit into the base unit, so that even if the glass tip broke the $NO_2$ would be contained; 3) an interlock so that the disposable unit can only be used once; 4) warnings and alarms, including, but not limited to, warning lights for low battery, low or high NO, wrong flow, etc.; 5) an encased liquid reservoir, where the reservoir can be entirely encased in an activated charcoal sheath which will be of sufficient mass to adsorb all of the $NO_2$ in the storage vessel; 6) a thermal fuse on the heater element so that the unit can never exceed its set temperature; and 7) sensors for flow, pressure atmospheric pressure, etc.

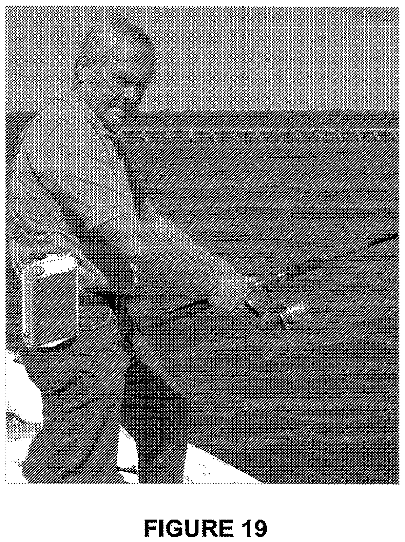

FIG. 19 shows the size of an exemplary device, in which a man is shown wearing the device while fishing. The miniaturization can be an important feature. Current commercially available delivery systems for inhaled NO can require a patient to be confined to a bed in a hospital and usually in an Intensive Care Unit. The ability to supply inhaled NO chronically in a simple fashion represents a breakthrough in treatment with inhaled NO.

**[0145]** A system can be relatively small. The system can weigh less than 64 ounces, less than 32 ounces or less than 16 ounces. The system can be less than 2 feet, less than 1.5 feet, less than 1 foot in height. The system can be less than 2 feet, less than 1.5 feet, less than 1 foot, less than 9 inches or less than 6 inches in width. The system can be less than 6 inches, less than 4 inches, less than 3 inches or less than 2 inches in depth.

A method of for delivering nitric oxide can include breaking the seal on a second end or opening a closed second end of a restrictor. The restrictor can have a first end in a reservoir containing a nitrogen dioxide source. The method can also include heating the reservoir and the restrictor, which can also heat the nitrogen dioxide source in the reservoir and nitrogen dioxide gas in the reservoir and/or the restrictor. As the nitrogen dioxide gas is heated, vapor pressure can accumulate within the reservoir, releasing the nitrogen dioxide gas into the restrictor. Once the second end is opened or unsealed, nitrogen dioxide gas that is forced into the restrictor can pass through the second end of the restrictor. The method can further include passing a gas from a gas supply across a second end of a restrictor. Passing gas from a gas supply across the second end of a restrictor can create negative pressure at the second end of the restrictor. The increased vapor pressure in the reservoir and/or the negative pressure at the second end of the restrictor can force $NO_2$ vapor through the restrictor. This can result in the $NO_2$ gas mixing with the gas from the gas supply. The $NO_2$ gas mixed with the gas from the gas supply can then be passed through at least one converter. Additionally, a method can include monitoring the level of NO with a NO sensor, monitoring the level of $NO_2$ with a $NO_2$ sensor, or monitoring the temperature with a temperature sensor.

In one example, the system is activated by breaking the seal of a sealed restrictor, for example, breaking off the tip of a quartz capillary restrictor tube. $NO_2$ vapor can be expelled from the reservoir at a constant flow rate, which can be dependent on the availability of liquid in the reservoir and the temperature of the reservoir. The $NO_2$ vapor can mix with gas, e.g. air, from a small pump and the dilute $NO_2$ mixture can then be allowed to pass through a first converter, where the $NO_2$ can be converted into NO. The converter can be made up of fine silica gel soaked in a reducing agent, e.g. ascorbic acid solution, and then partially dried. The NO in gas stream can be flowed to the second converter. A second converter can provides double redundancy. Each of the two cartridges can have sufficient silica gel-ascorbic acid powder to convert 1.5 times the content of the liquid in the reservoir. Also, each cartridge can be manufactured from a different lot. The NO in gas stream can be passed across an optional NO, an optional $NO_2$ electrochemical sensor, an optional pressure and/or optional flow sensor. The NO vapor in air can then be delivered to a patient by means of a nasal cannula.

For home use, patients can use a system that delivers a fixed output per unit time. A patient needing a high dose can be provided with a modified system in which increased output can be achieved either by increasing the temperature of the reservoir, changing the diameter of the restrictor or length of the restrictor.

In a hospital setting, the nurse may have a need to vary both the flow rate of air and the gas concentration. This can be accomplished by varying the temperature of the reservoir for increase the output of the reservoir. The air flow can be adjusted, either from a compressor or from increasing the power of a small built in air pump. A system with variable flow and variable output can include a monitor and display of the flow rate and the NO concentration.

A small liquid source of dinitrogen tetroxide ($N_2O_4$) in combination with a cartridge can open up a wide variety of medical applications to the treatment of inhaled nitric oxide (NO). The key enabling technology is the relatively small and light weight nitrogen dioxide/nitric oxide source that can provide gas for days without the need of a gas bottle, gas regulators, monitors, etc. Furthermore, a device as described can run on batteries, and therefore, can require minimal to no electronics. The mobility provided by the small size and minimal electronics can make it possible to for a patient to be out of a hospital bed, typically a hospital bed in an intensive care unit. This, in turn, can permit a patient to go home or go back to work while still receiving nitric oxide continuously. The cost saving for the nitric oxide alone can be very large, in addition to possible savings which can result from coming off a very expensive ventilator. Use of nitric oxide outside a hospital setting could also free up space in the intensive care unit or in the hospital ward. The cost reduction for use of the equipment alone can be from approximately $3,000 per day down to $300 per day.

The relatively small and inexpensive devices can also have applications to animals. For animals, such as cattle and horses, the small size of the device and ability to use the device in non-hospital settings can make the difference between keeping the animal alive or not.

DELIVERY:

As mentioned above, the devices described herein can be used to treat patients. One method or treating a patient can include delivering an effective concentration of the nitric oxide to the patient.

The term "effective concentration" means the concentration of nitric oxide that will elicit the biological or medical response of a tissue, system, animal or human that is being sought by a researcher or clinician. It is a concentration that is sufficient to significantly affect a positive clinical response while maintaining diminished levels of side effects. The concentration of nitric oxide which may be administered to a subject in need thereof can be in the range of 1-100 ppm, or preferably 30-90ppm, for example, about 1 ppm, about 5 ppm, about 10 ppm, about 20 ppm, about 30 ppm, about 40 ppm, about 50 ppm, about 60 ppm, about 70 ppm, about 80 ppm, or about 90 ppm, administered in continuous or intermittent delivery. The concentration and delivery regimen (i.e. continuous or intermittent) of nitric oxide each can be selected in accordance with a variety of factors including type, species, age, weight, sex or medical condition of the patient, the severity of the condition to be treated, or the route of administration, or combinations thereof. The sensitivity or vulnerability of the patient to side effects can also be considered. An effective concentration can also be referred to as a dose or doseage.

While nitric oxide doses are commonly given as a concentration (ppm), delivery of nitric oxide can also be given in an effective amount. The term "effective amount" can mean the amount of nitric oxide that will elicit the biological or medical response of a tissue, system, animal or human that is being sought by a researcher or clinician. It is an amount that is sufficient to significantly affect a positive clinical response while maintaining diminished levels of side effects. The effective amount of nitric oxide which may be administered to a subject in need thereof can be in the range of 0.01 mg to 10 mg, or preferably 0.025 mg to 5 mg. The effective amount can be, for example, at least about 0.01 mg, at least about 0.025 mg, at least about 0.05 mg, at least about 0.075 mg, at least about 0.1 mg, at least about 0.15 mg, at least about 0.2 mg, at least about 0.5 mg, at least about 0.75 mg, at least about 1 mg, at least about 1.5 mg, at least about 2 mg, at least about 2.5 mg, at least about 3 mg, at least about 4 mg or at least about 5 mg administered in continuous or intermittent delivery. The effective amount can be, for example, at most about 50 mg, at most about 20 mg, at most about 15 mg, at most about 10 mg, at most about 7.5 mg, at most about 5 mg, at most about 2.5 mg, at most about 2 mg, at most about 1.5 mg, at most about 1 mg, at most about 0.5 mg, or at most about 0.1 mg administered in continuous or intermittent delivery. The amount and delivery regimen (i.e. continuous or intermittent) of nitric oxide each can be selected in accordance with a variety of factors including type, species, age, weight, sex or medical condition of the patient, the severity of the condition to be treated, or the route of administration, or combinations thereof. The sensitivity or vulnerability of the patient to side effects can also be considered. An effective amount can also be referred to as a dose or doseage.

In some cases, an effective amount can be given in the milligrams per kilogram of the patient administered per hour. For example, the effective amount can be, for example, at least about 0.01 mg/kg/hr, at least about 0.025 mg/kg/hr, at least about 0.05 mg/kg/hr, at least about 0.075 mg/kg/hr, at least about 0.1 mg/kg/hr, at least about 0.15 mg/kg/hr, at least about 0.2 mg/kg/hr, at least about 0.5 mg/kg/hr, at least about 0.75 mg/kg/hr, at least about 1 mg/kg/hr, at least about 1.5 mg/kg/hr, at least about 2 mg/kg/hr, at least about 2.5 mg/kg/hr or at least about 5 mg/kg/hr administered in continuous or intermittent delivery. The amount can be, for example, at most about 50 mg, at most about 10 mg/kg/hr, at most about 7.5 mg/kg/hr, at most about 5 mg/kg/hr, at most about 2.5 mg/kg/hr, at most about 2 mg/kg/hr, at most about 1.5 mg/kg/hr, at most about 1 mg/kg/hr, at most about 0.5 mg/kg/hr, at most about 0.1 mg/kg/hr or at most about 0.05 mg/kg/hr administered in continuous or intermittent delivery. The milligram per kilogram per hour amount and delivery regimen (i.e. continuous or intermittent) of nitric oxide each can be selected in accordance with a variety of factors including type, species, age, weight, sex or medical condition of the patient, the severity of the condition to be treated, or the route of administration, or combinations thereof. The sensitivity or vulnerability of the patient to side effects can also be considered. An effective amount expressed as in milligrams per kilogram of the patient administered per hour can also be referred to as a dose or doseage.

Nitric oxide can be delivered to a patient between at least 15 minutes, at least 30 minutes, at least 45 minutes, at least 1 hour, at least 2 hours, at least 3 hours, at least 4 hours or at least 6 hours, or at most 6 hours, at most 4 hours, at most 3 hours, at most 2 hours, at most 1.5 hours, at most 1.25 hours or at most 1 hour after a trauma or injury. Preferably, nitric oxide can be delivered to a patient between about 15 minutes and about 3 hours, between about 30 minutes and 2 hours or between about 45 minutes and about 1.25 hours after a trauma or injury.

Nitric oxide can be delivered to a patient continuously. Alternatively, nitric oxide can be delivered to a patient intermittently. Intermittently can mean that nitric oxide can be delivered to a patient for a first period of time, delivery can then be terminated for a second period of time, and then nitric oxide can be delivered to a patient for a third period of time. In other words, intermittently means that the nitric oxide is delivered and then temporarily delivery of nitric oxide is reduced or stopped before resuming delivery again. The first, second and third periods of time can be equivalent periods of time or different periods of time. A period of time can be less than 1 second, less than 2 seconds, less than 5 seconds, less than 10 seconds, less than 15 seconds, less than 20 seconds, less than 30 seconds, less than 45 seconds, less than 1 minute, less than 5 minutes, less than 10 minutes, less than 15 minutes, less than 20 minutes, less than 30 minutes, less than 1 hour, less than 2 hours, less than 5 hours, less than 6 hours, less than 9 hours, less than 12 hours or less than 24 hours. A period of time can be greater than 1 second, greater than 2 seconds, greater than 5 seconds, greater than 10 seconds, greater than 15 seconds, greater than 20 seconds, greater than 30 seconds, greater than 45 seconds, greater than 1 minute, greater than 5 minutes, greater than 10 minutes, greater than 15 minutes, greater than 20 minutes, greater than 30 minutes, greater than 1 hour, greater than 2 hours, greater than 5 hours, greater than 6 hours, greater than 9 hours, greater than 12 hours or greater than 24 hours.

Intermittently can also mean the nitric oxide is delivered to a patient in pulses, such as a pulse of nitric oxide per inhalation by the patient, every other inhalation by the patient or every third inhalation by the patient.

Nitric oxide can be delivered to a patient for a period of time. The period of time includes the delivery over a treatment session. For example, if nitric oxide is delivered in half second pulses for 12 hours, the treatment session can be 12 hours, and therefore, the period of time the nitric oxide is delivered can be considered 12 hours not one half second. As a second example, if nitric oxide is delivered intermittently in 5 minute intervals for 12 hours, the treatment session can be 12 hours, and therefore, the period of time the nitric oxide is delivered is considered 12 hours not 5 minutes. Nitric oxide can be delivered to the patient for a period of at least 15 minutes, at least 30 minutes, at least 45 minutes, at least 1 hour, at least 2 hours, at least 4 hours, at least 6 hours, at least 8 hours, at least 12 hours, at least 24 hours, at least 36 hours or at least 48 hours, or at most 15 minutes, at most 30 minutes, at most 45 minutes, at most 1 hour, at most 2 hours, at most 4 hours, at most 6 hours, at most 8 hours, at most 12 hours, at most 24 hours, at most 36 hours or at most 48 hours.

Delivery of nitric oxide to the patient can be made through a patient interface. A patient interface can include a mouth piece, nasal cannula, face mask, or fully-sealed face mask.

A delivery device can operate at continuous gas flows. Gas flows can be between about 0.5 and 7 L/min, for example, at about 0.5 L/min, at about 1 L/min, at about 1.5 L/min, at about 2 L/min, at about 2.5 L/min, at about 3 L/min, at about 3.5 L/min, at about 4 L/min, at about 4.5 L/min, at about 5 L/min, at about 5.5 L/min, at about 6 L/min, at about 6.5 L/min, or at about 7 L/min. In a preferred embodiment, gas flow can be at about 1L/min.

A device or system can deliver nitric oxide gas in another gas, for example, air. If there is a need to have supplemental oxygen as well, a lumen of a cannula can be used to deliver oxygen. Alternatively, a dual lumen cannula can be used that has two tubes at each nostril, one for oxygen and one for NO. It can be undesirable to deliver the NO in 90% oxygen for two reasons: First, the oxygen can increase the rate of formation of $NO_2$ in the cannula by a factor of 5. This can be undesirable because the resulting $NO_2$ that can be formed is extremely toxic. Second, NO generally is not be delivered in 90% oxygen to avoid any possibility of a flammability hazard by passing oxygen through an ambulatory sample system.

Another option can be to use an oxygen conserver. Oxygen conservers are known in the art. Oxygen conservers can work by sensing when a breath is about to occur and then delivering a bolus of gas during some part of the inhalation cycle. Conservers can be designed to provide one or more oxygen pulses that coincide with breathing, which can include turning on the oxygen while a person inhales and turning it off when a person exhales. For example, a conserver can be used by COPD patients that need supplemental oxygen as they walk, climb stairs or perform other daily activities. The conservers can be designed to dole out a set oxygen dose each time a person inhales. This can be advantageous because it can allow the oxygen supply in a cylinder or bottle to last longer than it would if the oxygen flow were continuous.

A conserver can also be used with the nitrogen dioxide or nitric oxide source. A method for conserving liquid NO in a device can include utilizing a conserver. This can include stopping the flow of NO during exhalation and releasing a dose of NO during inhalation. In other words, instead of the gas flowing continuously, the conserver can stop the flow during exhalation, thereby conserving the nitric oxide. This can allow for the nitrogen dioxide source in a device to last from 2 to 6 times longer than without a conserver. A conserver can be qualified to ensure that the surfaces and components and rubber and plastic parts that come into contact with the NO gas are compatible with nitric oxide.

In some situations, a ventilator can also be used in the delivery of nitric oxide to a patient. Traditionally, nitric oxide gas is delivered to a patient by means of a ventilator. The source of NO can be a pressurized cylinder of NO in nitrogen, with the NO concentration being about 800 ppm. The traditional nitric oxide delivery systems may not be suitable for delivery of nitric oxide with a ventilator for a couple reasons.

First, the NO gas is usually introduced to a gas delivery line prior to the ventilator. While this can give a uniform nitric oxide profile within a breath, the added time in the ventilator can make it unsuitable for use because of the formation of nitrogen dioxide ($NO_2$), during this time delay.

Second, the preferred method can be to measure the instantaneous flow by means of a hot wire flow meter, and use the instantaneous flow measurement to time a valve, which can allow nitric oxide into the system. This feedback loop can provide a near constant nitric oxide profile, as measured in volume to volume units of ppm, within a breath (0% to 150% of the mean nitric oxide value). The nitric oxide- time profile can be held constant when measured on a volume to volume basis (parts per million). However, as the breathing rate changes, the constant ppm can lead to a variable dose when measured in milligrams (mg).

Instead of using a source of NO gas in nitrogen, the devices and systems described herein can use $NO_2$ in air or oxygen. In particular, the devices using a liquid $N_2O_4$ as a source can be advantageous with a ventilator. As described above, the $N_2O_4$ reservoir can be heated and the $N_2O_4$ can be allowed to vaporize to $NO_2$. As the temperature is increased above 21°C, which is the boiling point of $NO_2$, the pressure above the liquid can be increased above atmospheric pressure. The pressure differential can then be harnessed to expel $NO_2$ gas from the reservoir through a narrow restriction. The rate of $NO_2$ mass flow through the restriction can be independent of the downstream pressure as long as the pressure differential is greater than 2:1. The constant mass flow of $NO_2$ can then be diluted with air or oxygen.

Accordingly, for a ventilator, one advantage of a liquid source over storing the $NO_2$ gas in a gas bottle can be that the liquid source, can deliver a constant mass of $NO_2$ gas per unit time, irrespective of the dilution flow. This can be important because drugs can be delivered as milligrams per kilogram body weight, not in parts per million on a volume to volume basis. For example, if the number of breaths per minute increases, the liquid source can continue to deliver a constant milligram dose, although the concentration as measure in ppm can be diminished. On the other hand, if a gas bottle were used, then the dose can be typically adjusted to give constant ppm, which could actually increase the mg dose as the number of breaths increased.

Another advantage can be the reduction of the bulky and heavy high pressure gas bottles in the cramped space of an Intensive care unit

Still another advantage can be the elimination of risks and hazards associated with high pressure gas bottles and their propensity to leak. A typical gas bottle can be pressurized to above 2000 psi, and can be considered empty when the pressure falls below about 150 to 200 psi. The liquid source operating at 50 °C can only be pressurized to about 50 psi and can therefore inherently be a much safer system.

Vials of liquid can be advantageous because they can be stored in a pharmacy or small shelf as compared to outdoor storage of large gas bottles.

Gas bottles can be rented and there can be a daily rental fee that can be added to the cost of the gas. The gas bottles have to be tracked, and empty bottles returned for cleaning and refilling. By comparison, the liquid source can be a tiny disposable item that can be disposed of after use and not tracked or returned, which can be advantageous.

INJURIES, DISEASES AND CONDITIONS:

NO can be useful for the treatment of a number of injuries, diseases and conditions. The NO delivery devices described herein offer a novel way to treat these injuries, diseases and conditions because the NO delivery devices can include minimal or no electronics and monitors, and therefore, may not be limited to use a medical facility setting. This allows treatments of injuries, diseases and conditions in which treatment with NO was previously limited or non-existent. The delivery devices also allow NO to be delivered in dosage amounts, or concentrations, or modalities (pulsed or continuous delivery). Exemplary injuries, diseases and conditions that can be treated by delivering NO as described above and using the devices described herein are described in greater detail below.

PATIENT WHO HAS BEEN ADMINISTERED CARDIOPULMONARY RESUSCITATION (CPR)

Nitric oxide may also be able to help patients who have just undergone cardiopulmonary resuscitation ("CPR"). CPR can be administered to a person who has suffered cardiac arrest in an attempt to delivery oxygenated blood to the brain and heart, keeping these organs alive.

Patients who have just undergone cardiopulmonary resuscitation can have low blood pressure. Because of the low blood pressure, these patients may not be able to use many vasodilators or nitric oxide donors. However, inhaled nitric oxide can be used successfully with these patients.

One method of treating a patient can include delivering an effective concentration of the nitric oxide to the patient. Delivery of the nitric oxide can occur after cardiopulmonary resuscitation has been performed on the patient.

Nitric oxide can be delivered to a patient between at least 15 minutes, at least 30 minutes, at least 45 minutes, at least 1 hour, at least 2 hours, at least 3 hours, at least 4 hours or at least 6 hours, or at most 6 hours, at most 4 hours, at most 3 hours, at most 2 hours, at most 1.5 hours, at most 1.25 hours or at most 1 hour after cardiopulmonary resuscitation has been performed. Preferably, nitric oxide can be delivered to a patient between about 15 minutes and about 3 hours, between about 30 minutes and 2 hours or between about 45 minutes and about 1.25 hours after cardiopulmonary resuscitation has been performed.

The effective amount or effective concentration administered to treat a patient who has suffered an ischemic/reperfusion injury or an event resulting in inflammation in the central nervous system can be selected from the dosing and delivery described above. Further, any of the devices described above can be utilized for treating a patient who has suffered an ischemic/reperfusion injury or an event resulting in inflammation in the central nervous system.

SLEEP APNEA

Obstructive sleep apnea (OSA) can be associated with increased prevalence of cardiovascular and cerebrovascular morbidity. NO may play an important role in the regulation of blood pressure in OSA. The long-term complications, namely hypertension, myocardial infarction, and stroke, might be due to the repeated temporary dearth of NO in the tissues, secondary to a lack of oxygen, one of NO's two essential substrates. (See Nitric oxide (NO) and obstructive sleep apnea (OSA), Sleep Breath, 2003 Jun, 7(2):53-62, which is incorporated by reference in its entirety).

Circulating NO can be suppressed in OSA, and this is promptly reversible with the use of nasal Continuous Positive Airway Pressure (nCPAP). Nitric oxide can be one of the mediators involved in the acute hemodynamic regulation and long-term vascular remodelling in OSA. (See Circulating Nitric Oxide Is Suppressed in Obstructive Sleep Apnea and Is Reversed by Nasal Continuous Positive Airway Pressure, Am. J. Respir. Crit. Care Med., Volume 162, Number 6, December 2000, 2166-2171, which is incorporated by reference in its entirety).

The effective amount or effective concentration administered to a patient to treat sleep apnea can be selected from the dosing and delivery described above, for example, in a manner that supplies additional NO to the tissues.

In some embodiments, CPAP machines can provide pressurized air that is forced into the lungs. Since the air is forced in through the mask, the body's naturally produced NO in the nasal passages can be by passed and the lungs would therefore not receive the low level NO source, which can be, for example, approximately 0.05 ppm. For this application, a small ambulatory cartridge can be used to supply NO gas into the air being forced into the lungs. The time period for supplying the NO gas can be for at least 4 hours, at least 6 hours, at least 8 hours or at most 12 hours, at most 10 hours, or at most 8 hours, for instance, during sleeping. Most preferably, the time period for supplying the NO gas can be between 6 to 10 hours.

In some embodiments, if the patient is using oxygen instead of air, then the NO can be added to the oxygen flow to the CPAP mask. The concentration of NO can be at least 1 ppm, at least 5 ppm, at least 10 ppm or at least 15 ppm. The concentration of NO can be at most 30 ppm, at most 25 ppm, at most 20 ppm, at most 15 ppm, at most 10 ppm or at most 5ppm. The concentration of NO can be in the 1 to 5 ppm range, or possibly as high as 10 to 20 ppm. Other modalities that may prove useful depending upon the condition of the patient are to start with a high dose, of say 20 to 80 ppm and then reduce the dose down to the low ppm range.

Any of the devices described above can be utilized for treating sleep apnea.

Idiopathic Pulmonary Fibrosis (IPF)

IPF disease is estimated to kill more women than breast cancer. There is no known treatment for IPF. Impaired gas exchange leading to hypoxemia in IPF can be driven by ventilation/perfusion abnormalities and intrapulmonary shunts. Selective vasodilation of better ventilated segments by inhaled NO can improve oxygenation, and small vessel reversibility may persist into late stages of the disease. Inhaled NO can improve oxygenation in the setting of IPF with superimposed pulmonary hypertension. (See Outpatient Inhaled Nitric Oxide in a Patient with Idiopathic Pulmonary Fibrosis: A Bridge to Lung Transplantation, Journal of Heart Lung Transplantation, 2001, 20:1224-1227, which is incorporated by reference in its entirety).

The effective amount or effective concentration administered to a patient to treat IPF can be selected from the dosing and delivery described above.

Any of the devices described above can be utilized for treating IPF.

PULMONARY ARTERIAL HYPERTENSION

Pulmonary Arterial Hypertension (PAH) can be associated with a defect in the production of nitric oxide (NO) and with decreased NO induced vasodilatation. This deficit can be indirectly addressed via the use of PDE-5 inhibitors. Inhaled nitric oxide can selectively dilate pulmonary vasculature in adult patients with pulmonary hypertension, irrespective of etiology.

Chronic delivery of inhaled NO to ambulatory patients with PPH can lead to improvement, in some cases significant. (See Channick, R. N., J. W. Newhart, et al., "Pulsed delivery of inhaled nitric oxide to patients with primary pulmonary hypertension: an ambulatory delivery system and initial clinical tests," Chest, (1996), 109(6): 1545-1549; Perez-Penate, G. M., G. Julia-Serda, et al., "Long-term inhaled nitric oxide plus phosphodiesterase 5 inhibitors for severe pulmonary hypertension," J Heart Lung Transplant, (2008), 27(12): 1326-1332, Epub 2008 Oct 1326; ClinicalTrials.gov Identifier: NCT00352430, each of which is incorporated by reference in its entirety).

Inhaled NO, either alone or in combination with a PDE5 inhibitor, can be a potential long-term treatment option for severe pulmonary hypertension. Other delivery systems have included gas bottles and the patients were tethered to the gas bottles in their home. Use of a gas bottle delivery system includes considerable logistics to assure there are enough gas bottles. Additionally, the cost and complexity of using gas bottles can be great. A gas bottle, when empty, may need to be changed out, the regulators may need to be disconnected, and then the regulators may need to be re-attached to the new gas bottle. Gas bottles can hinder use in the home of an NO treatment plan due to the prohibitive cost ($3,000 per day), complexity, safety, and logistics. In addition, the chemical monitors that are needed to operate the systems may need to be calibrated daily. Calibration can involve additional gas bottles and specialized calibration equipment. Nevertheless, NO can be an effective in treating the PAH of these patients.

Any of the devices described above can be utilized for treating PAH. The delivery devices described above can make it possible to treat patients. There has been a need in the field for a practical way to use inhaled NO for 24 - 7 use. The devices can be used all day every day. For devices with batteries, the battery power can allow the user to be fully ambulatory. In embodiments with liquid storage of $N_2O_4$, the liquid storage vessel can be designed to last 24 hours, so that the user can use a new cartridge every day. The liquid $N_2O_4$ can vaporize to $NO_2$, which can then be chemically reduced to NO over ascorbic acid. The delivery to the patient can be by means of a nasal cannula. For those patients that also require supplemental oxygen, the oxygen can be supplied by a dual lumen cannula, where the oxygen supply is from a conventional oxygen storage vessel or oxygen generator.

The effective amount or effective concentration administered to a patient to treat PAH can be selected from the dosing and delivery described above.

Pulmonary Hypertension Associated With COPD

Pulmonary hypertension can be a frequent complication of severe chronic obstructive pulmonary disease (COPD) and, as a form of secondary PH, can be amenable to the vasodilatory effect of inhaled NO. (See Kumar Ashutosh, Kishor Phadke, Jody Fragale Jackson, David Steele, Use of nitric oxide inhalation in chronic obstructive pulmonary disease, Thorax 2000, 55:109-113; K Vonbank, R Ziesche, T W Higenbottam, L Stiebellehner, V Petkov, P Schenk, P Germann, L H Block, Controlled prospective randomised trial on the effects on pulmonary haemodynamics of the ambulatory long term use of nitric oxide and oxygen in patients with severe COPD, Thorax, 2003, 58:289-293, each of which is incorporated by reference in its entirety).

Administration of NO with oxygen can result in improvements in hemodynamics in COPD patients over a 3 month period when compared to oxygen alone. Additionally, administration of NO with oxygen did not result in a decrease in oxygenation. Therefore, nitric oxide together with oxygen may be safely and effectively used for the long term treatment of PAH associated with COPD.

As with PAH above, the use of any of the described devices can simplify the treatment of this class of patients. Depending on the severity of the disease, a fraction of the patients may no longer require supplemental oxygen.

The effective amount or effective concentration administered to a patient to treat PAH associated with COPD can be selected from the dosing and delivery described above.

Chronic Obstructive Pulmonary Disease (COPD)

In hypoxic lung diseases, including severe COPD, endothelial release of NO can be impaired. Inhaled NO can have an impact on gas exchange in severe COPD with various degrees of pulmonary arterial pressure elevation, even in cases without frank pulmonary hypertension. In patients with COPD and secondary pulmonary arterial hypertension, inhaled NO for three months can decrease pulmonary artery pressures and pulmonary vascular resistance, and can increase cardiac index with no negative effects observed.

The effective amount or effective concentration administered to a patient to treat COPD can be selected from the dosing and delivery described above.

Any of the devices described above can be utilized for treating COPD.

Pulmonary Hypertension Associated With Idiopathic Pulmonary Fibrosis (IPF)

Pulmonary arterial hypertension can be a feature of later stage disease and indicator of poor prognosis in IPF. Low-dose inhalation of nitric oxide (NO) can improve pulmonary haemodynamics and gas exchange in patients with stable idiopathic pulmonary fibrosis (IPF). Combined NO and oxygen inhalation can improve pulmonary hemodynamics and increased arterial oxygenation. (See Yoshida et al., The effect of low-dose inhalation of nitric oxide in patients with pulmonary fibrosis, Eur Respir J, (1997) 10:2051-4, which is incorporated by reference in its entirety). Inhaled nitric oxide maintained ventilation perfusion matching and decreased pulmonary vascular resistance without a decrease in arterial oxygen tension. (See Ghofrani et al., Sildenafil for treatment of lung fibrosis and pulmonary hypertension: a randomised controlled trial, Lancet, (2002) 360:895-900, which is incorporated by reference in its entirety).

The effective amount or effective concentration administered to a patient to treat PAH associated with IPF can be selected from the dosing and delivery described above. Further, any of the devices described above can be utilized for treating PAH associated with IPF.

SICKLE CELL DISEASE-RELATED CONDITIONS, INCLUDING PULMONARY HYPERTENSION (PH), SICKLE CELL CRISIS (SCC) AND ACUTE CHEST SYNDROME (ACS)

Nitric oxide may be used in the treatment of sickle cell disease. Research and treatment in this field is typically done in a clinic using ventilator based equipment because it is the only technology available. The use of the liquid source ambulatory platform can allow a patient to use nitric oxide in their home, without the need for special equipment. An advantage of using a liquid source ambulatory platform, or any other device described above, would be the reduction in the cost of the treatment by minimizing hospital visits and stays in expensive intensive care settings. The drug could also be used prophylactically to prevent a crisis. The availability of a viable delivery system can result in an effective treatment option.

Pulmonary hypertension (PH) can be complication of Sickle Cell Disease (SCD) and can be driven by proliferative vasculopathy, in situ thrombosis, and vascular dysfunction related to NO scavenging by free hemoglobin generated via intravascular hemolysis. NO can act on all three components contributing to PH in SCD. Treatment of SCD patients by sildenafil, a NO-generating agent, can reduce pulmonary pressures in patients with SCD and PH and can also decrease platelet activation, which has been proposed to provide an additional benefit in terms of preventing PH progression. (See Platelet Activation in Patients with Sickle Cell Disease, Hemolysis-Associated Pulmonary Hypertension, and Nitric Oxide by Cell-Free Hemoglobin, Blood, 2007 Sep 15, 110(6): 2166-72, which is incorporated by reference in its entirety).

Alterations in the levels of 1-arginine and nitric oxide metabolite levels observed in children with SCD at baseline and during sickle cell crisis (SCC) suggest a relationship between the 1-arginine-nitric oxide pathway and vaso-occlusion in SCD. This, in turn, can be treated via inhaled NO supplementation. NO can produce significant reductions in opiate use pain score by visual analog scale and non-significant reductions in length of stay. (See Patterns of Arginine and Nitric Oxide in Patients with Sickle Cell Disease with Vaso-Occlusive Crisis and Acute Chest Syndrome, Journal of Pediatric Hematology/Oncology, 2000 Dec, 22(6):515-20; Chronic Sickle Cell Lung Disease: New Insights into the Diagnosis, Pathogenesis and Treatment of Pulmonary Hypertension British, Journal of Hematology, 2005, 129:449-464; Platelet Activation in Patients with Sickle Cell Disease, Hemolysis-Associated Pulmonary Hypertension, and Nitric Oxide by Cell-Free Hemoglobin, Blood, 2007 Sep 15, 110(6): 2166-72; Preliminary Assessment of Inhaled Nitric Oxide for Acute Vaso-occlusive Crisis in Pediatric Patients with Sickle Cell Disease, JAMA, 2003, 289:1136-1142, each of which is incorporated by reference in its entirety).

Acute Chest Syndrome (ACS) can also be treated with inhaled nitric oxide. (See Nitric Oxide Successfully Used to Treat Acute Chest Syndrome of Sickle Cell Disease in a Young Adolescent, Critical Care Medicine, 1999 Nov, 27, (11):2563-8, which is incorporated by reference in its entirety).

The effective amount or effective concentration administered to a patient to treat sickle cell-disease related conditions can be selected from the dosing and delivery described above. Further, any of the devices described above can be utilized for treating sickle cell-disease related conditions.

ALPHA-1-ADRENORECEPTOR VASOREACTIVITY IN CHRONIC KIDNEY DISEASE

The lack of availability of a system that can be used to treat the patient with nitric oxide outside the confines of an Intensive Care Unit can limit research and treatment of alpha-1-adrenoreceptor in vasoreactivity in chronic kidney disease.

The overall production of nitric oxide (NO) can be decreased in chronic kidney disease (CKD) which contributes to cardiovascular events and further progression of kidney damage. Patients with chronic kidney disease (CKD) can have high blood pressure and can be at high risk for cardiovascular disease. Low availability of NO may be responsible for high activity of alpha 1-adrenoceptor system in patients with CKD (role of vascular nitric oxide in regulating alpha-adrenergic vasoreactivity). Interventions that can restore NO production by targeting these various pathways are likely to reduce the cardiovascular complications of CKD as well as slowing the rate of progression.

Any of the devices described above can be utilized for treating alpha-1-adrenoreceptor in vasoreactivity in chronic kidney disease. For example, NO can be delivered to these patients with the use of an ambulatory liquid source system. (See Nitric oxide deficiency in chronic kidney disease, Am J Physiol Renal Physiol, 294:F1-F9, 2008; Clinical-Trials.gov Identifier: NCT00240058, each of which is incorporated by reference in its entirety).

The effective amount or effective concentration administered to a patient to treat alpha-1-adrenoreceptor in vasoreactivity in chronic kidney disease can be selected from the dosing and delivery described above.

INFECTIOUS LUNG DISEASES

Treatment of infectious lung diseases with NO takes advantage of the antibacterial and antiviral properties of nitric oxide. NO can be used in the ICU with a Ventilator for the treatment of infectious lung diseases as a last resort. The ambulatory system can make it viable to use NO on patients who are very sick, but not necessarily hospitalized. The treatment can be effective against all types of bacterial and viral lung infections, of which TB and Influenza are but an example.

Tuberculosis (TB)

Nitric oxide (NO) can be important in host defense against *Mycobacterium tuberculosis.* Adjuvant-inhaled NO can be delivered to patients with pulmonary tuberculosis. For example, it has been previously demonstrated that NO can be administered at 80ppm can be safely delivered to patients with pulmonary tuberculosis. (See What is the role of nitric oxide in murine and human host defense against tuberculosis? Am. J. Respir. Cell. Mol. Biol. 25:606-612.)-1, The Proceedings of the American Thoracic Society 3:161-165 (2006); Inhibition of Respiration by Nitric Oxide Induces a Mycobacterium tuberculosis Dormancy Program Nitric Oxide 2006 February, 14 (1): 21-9; Inhaled Nitric Oxide Treatment of Patients with Pulmonary Tuberculosis Evidenced by Positive Sputum Smears Antimicrobial Agents and Chemotherapy, March 2005, p. 1209-1212, Vol. 49, No. 3, each of which is incorporated by reference in its entirety).

Any of the devices described above can be utilized for treating TB. For example, NO can be delivered to these patients with the use of an ambulatory liquid source system.

The effective amount or effective concentration administered to a patient to treat TB can be selected from the dosing and delivery described above.

Influenza

Nitric oxide (NO) can play an important role in host defense through its potent antiviral properties. The ability of NO to inhibit viral replication and reduce the pro-inflammatory consequences of such infections may suggest that administration of NO can be of broad therapeutic utility in viral infections. Unlike vaccines, which are designed for specific viral strains such as H1N1, inhaled NO may be universally effective against all influenza strains, presenting a significant breakthrough in the control of viral pandemics. Nitric oxide can also be a viable therapeutic approach for viral exacerbations of airway diseases.

Inhaled NO for can prevent the growth of the influenza virus. Inhaled Nitric Oxide can also be utilized as a rescue therapy in critically ill patients with influenza A (H1N1) infection. (See Nitric oxide inhibits interferon regulatory factor-1 and nuclear factor-kB in rhinovirus infected epithelial cells, J. Allergy Clin. Immunol, 2009, in press; Role of nasal nitric oxide in the resolution of experimental rhinovirus infection, J. Allergy Clin. Immunol., 2004, 113:697-702; Critically ill patients with 2009 influenza A(H1N1) infection in Canada, JAMA, 2009, Nov 4, 302(17):1872-9, Epub 2009 Oct 12, each of which is incorporated by reference in its entirety).

The effective amount or effective concentration administered to a patient to treat influenza can be selected from the dosing and delivery described above. Further, any of the devices described above can be utilized for treating influenza.

EFFECT OF TOBACCO SMOKE

The nitric oxide levels in cigarette smoke increase from about 50 ppm for the first puff to over 2000 ppm for the last puff. One reason for the increase is that the NO can be formed in the flame front from nicotine and organic and inorganic nitrates that are present in the tobacco. These organics can distill ahead of the glowing hot zone and the concentration in the remaining unburned tobacco can build up as the cigarette is smoked. Anecdotally, habitual cigarette smokers reach for a cigarette to help "clear" their mind, which, without committing to any theory, may imply that the very high NO levels in the smoke are having an impact of the brain-neuron chemistry. This may suggest that part of the addiction of cigarettes can be due to the nitric oxide. Thus, a possible weaning approach can be to inhale a single breath of NO at a relatively high concentration of NO, for example, at least 50 ppm, at least 100 ppm, at least 150 ppm, at least 200 ppm, at least 500 ppm, at least 750 ppm, at least 1000 ppm, at least 1500 ppm or at least 2000 ppm of NO. This breath (which can also be called a pulse) can be followed by at least 5, at least 7, at least 10, at least 15, at least 20 or at least 25 breaths of room air. Preferably, it is followed by between 5 and 25 breaths of room air, and most preferably, between 10 and 20 breaths of room air. The single breath of NO followed by the breaths of room air can be repeated. For example, the pattern can be repeated at least 2 times, at least 4 times, at least 5 times, at least 8 times, at least 10 times or at least 15 times. The pattern can be repeated most preferably at least 8 times.

Any of the devices described above can be utilized for treating a patient who is attempting to quit smoking. However, an alternative would be to configure a delivery device described herein in the format of a cigarette. A person could inhale a microgram amount of nitric oxide into the lungs, much like a cigarette. An amount can be at least about 0.01 mg, at least about 0.025 mg, at least about 0.05 mg, at least about 0.075 mg, at least about 0.1 mg, at least about 0.15 mg, at least about 0.2 mg, at least about 0.5 mg, at least about 0.75 mg, at least about 1 mg, at least about 1.5 mg, at least about 2 mg, at least about 2.5 mg, at least about 3 mg, at least about 4 mg or at least about 5 mg of NO. An alternative approach would be to burn a simple material, such as paper, that had been soaked in a material that was high in organic nitrogen (amino acids) or inorganic nitrogen (nitrates). This could also be used as a very simple and cheap method to deliver inhaled nitric oxide to patients a patient who is attempting to quit smoking.

The effective amount or effective concentration administered to a patient who is attempting to quick smoking can be selected from the dosing and delivery described above.

EFFECT OF ON NEURONS

The discovery of nitric oxide (NO) as a neurotransmitter has altered thinking about synaptic transmission. Being a labile, free radical gas (though in most biological situations NO is in solution), NO may not be stored in synaptic vesicles. Instead, NO can be synthesized as needed by NO synthase (NOS) from its precursor L-arginine. Rather than exocytosis, NO can diffuse from nerve terminals. NO may not react with receptors but diffuses into adjacent cells. In place of reversible interactions with targets, NO can form covalent linkages to a multiplicity of targets which can include enzymes, such as guanylyl cyclase (GC) or other protein or nonprotein targets.

Inactivation of NO can involve diffusion away from targets as well as covalent linkages to an assortment of small or large molecules such as superoxide and diverse proteins. NO can influence neurotransmitter release.

Most neurons in the mammalian brain can be produced during embryonic development. However, several regions of the adult brain can continue to spawn neurons through the proliferation of neural stem cells. These new neurons can be integrated into existing brain circuitry.

Nitric oxide can be a pivotal, natural regulator of the birth of new neurons in the adult brain. Blocking nitric oxide production can stimulate neural stem cell proliferation and can dramatically increase the number of neurons that are generated in the brains of adult rats. Importantly, the new neurons that arise as a consequence of blocking nitric oxide production can display properties of early development neurons, and they can contribute to the architecture of the adult brain. Therefore, modulating nitric oxide levels can be an effective strategy for replacing neurons that are lost from the brain due to stroke or chronic neurodegenerative disorders such as Alzheimer's, Parkinson's, and Huntington's disease.

The effective amount or effective concentration administered to a patient to treat neurodegenerative disorders can be selected from the dosing and delivery described above. Further, any of the devices described above can be utilized for treating a patient who has a neurodegenerative disorder.

Acute Hypoxemic Respiratory Failure (AHRF)

Inhaled NO widely can be used in the neonatal intensive care unit and can be a safe and effective agent in this setting, in addition to extensive clinical experience. Inhaled NO can be considered a standard therapy in neonates with AHRF and can improve short term oxygenation in older children with AHRF and long term oxygenation in older children with severe hypoxemia and AHRF.

See Effects of Inhaled Nitric Oxide in the Treatment of Acute Hypoxemic Respiratory Failure (AHRF) in Pediatrics ClinicalTrials.gov Identifier: NCT00041561-Phase II; Pediatric Acute Hypoxemic Respiratory Failure: Management of Oxygenation J Intensive Care Med 2004; 19; 140, each of which is incorporated by reference in its entirety.

A patient with AHRF can be a newborn. A newborn, also referred to a neonate, can be patient who is less than 1 year old, less than 6 months old, less than 3 months old, less than 2 months old or less than 1 month old. Most preferably, a newborn is less than 3 months old. A newborn may or may not have been born after full gestation (e.g. 40 weeks gestation). For example, a newborn could have been born premature, for example, having a gestation period of less than 40 weeks, more specifically, less than 36 weeks. An infant can be a patient who is less than 1 year old.

The effective amount or effective concentration administered to a patient to treat AHRF can be selected from the dosing and delivery described above. Further, any of the devices described above can be utilized for treating an AHRF patient.

Respiratory Distress Syndrome (RDS)

Pulmonary vasoconstriction and hypoxemia can be prominent processes in RDS, and inhaled NO can produce improvements in oxygenation via selective pulmonary vasodilation. In animal models, inhaled NO can also reduce lung inflammation, can improve surfactant function, can attenuate hyperoxic lung injury and can promote lung growth. In randomized multi-center clinical trials, inhaled NO can demonstrate a reduction in the incidence of chronic lung disease (bronchopulmonary dysplasia) in preterm infants above 1000g in weight. Additionally, improvement in neuro-developmental outcomes among preterm infants can be seen treated with inhaled NO. (See Inhaled NO for Preterm Infants-Getting to Yes?, N Engl J Med, 2006 Jul 27, 355(4):404-406; Inhaled Nitric Oxide in Preterm Infants Undergoing Mechanical Ventilation, N Engl J Med., 2006 Jul 27, 355(4):343-54; Early Inhaled Nitric Oxide Therapy in Premature Newborns with Respiratory Failure, N Engl J Med., 2006 Jul 27, 355 (4):354-64; Preemie Inhaled Nitric Oxide Study. Inhaled nitric oxide for premature infants with severe respiratory failure, N Engl J Med., 2005 Jul 7, 353(1):13-22; Inhaled Nitric Oxide in Premature Infants with the Respiratory Distress Syndrome, N Engl J Med, 2003, 349:2099-107; Neuro-developmental Outcomes of Premature Infants Treated with Inhaled Nitric Oxide, N Engl J Med, 2005 Jul 7, 353(1):23-32, each of which is incorporated by reference in its entirety).

A patient with RDS can be a newborn. A newborn can be patient who is less than 1 year old, less than 6 months old, less than 3 months old, less than 2 months old or less than 1 month old. Most preferably, a newborn is less than 3 months old. A newborn may or may not have been born after full gestation (e.g. 40 weeks gestation). For example, a newborn could have been born premature, for example, having a gestation period of less than 40 weeks, more specifically, less than 36 weeks. An infant can be a patient who is less than 1 year old.

The effective amount or effective concentration administered to a patient to treat RDS can be selected from the dosing and delivery described above. Further, any of the devices described above can be utilized for treating a patient who has RDS.

Post Operative Cardiopulmonary Bypass, Cardiac Surgeries And Procedures

Pulmonary Hypertension (PH) can be a major risk factor for perioperative morbidity and mortality in patients during and after cardiac surgery employing cardiopulmonary bypass (CPB). Impaired right ventricular (RV) function due to elevated pulmonary vascular resistance (PVR) can make discontinuation of cardiopulmonary bypass (CPB) particularly laborious, and urgent re-initiation of CPB can sometimes be deemed necessary. Nitric oxide can be an effective agent for lowering pulmonary vascular resistance with resulting improvement in right ventricular function

Elevation of pulmonary pressures can occur in the postoperative setting following cardiopulmonary bypass; suggested mechanisms can include reduction in endogenous nitric oxide production, incomplete myocardial protection, release of vasoactive substances or unfavorable changes in ventricular loading conditions. In this setting, the use of a selective pulmonary vasodilator for hemodynamic support can be logical step.

Inhaled prostacyclin (iPGI2) and nitric oxide (iNO) can be used in patients affected by severe mitral valve stenosis and pulmonary hypertension. Mean pulmonary artery pressure and pulmonary vascular resistance can be decreased and cardiac indices and right ventricular ejection fraction can be increased in the iNO and iPGI2 patients. Patients using inhaled drug can be weaned easily from cardiopulmonary bypass (and can have a shorter intubation time and intensive care unit stay. CABG patients treated with inhaled NO can demonstrate improvement in oxygenation associated with reductions in pulmonary vascular resistance, with the effect most pronounced in patients with underlying pulmonary hypertension prior to surgery. (See Beneficial effects of inhaled nitric oxide in adult cardiac surgical patients, Ann Thorac Surg, 2002, 73:529-533; Treatment of pulmonary hypertension in patients undergoing cardiac surgery with cardiopulmonary bypass: a randomized, prospective, double-blind study, J Cardiovasc Med (Hagerstown), Feb 2006, 7(2):119-23; Response to nitric oxide during adult cardiac surgery, Journal of Investigative Surgery, 2002, 15 (1): 5-14; Dose response to nitric oxide in adult cardiac surgery patients, 2001, Journal of Clinical Anesthesia, 13(4): 281-60, each of which is incorporated by reference in its entirety.

The effective amount or effective concentration administered to a patient to treat cardiac conditions, such as those that occur following cardiac surgery, can be selected from the dosing and delivery described above. Further, any of the devices described above can be utilized for treating a patient who has a cardiac condition, such as a condition that occurs following cardiac surgery.

ISCEHMIC/REPERFUSION INJURIES

Nitric oxide can improve the outcome for a patient who has suffered from an ischemic (or hypoxic) injury and/or a reperfusion injury. Examples of these types of injuries can include injuries resulting from cardiac arrest, stroke, aneurism, strangulation, suffocation, hypothermia, respiratory trauma, and central nervous system trauma, including spinal cord trauma.

Central nervous system ("CNS") injuries and trauma encompass a wide variety of medical and traumatic insults to the brain and spinal cord. For example, stroke is the third leading cause of death in the developed world with one stroke occurring approximately every minute in the United States. Mortality rate is about 30% but more than 4 million stroke survivors are alive today, the majority of these individuals are left with varying degrees of disability. Clinical trials have yet to demonstrate therapeutic neuroprotection in ischemic stroke (i.e., stroke related to disruption of blood flow due to clot/thrombus formation) and spinal cord. Thrombolytic therapy (defined as use of an agent which causes dissolution or destruction of a thrombus) has many limitations, but it remains the only approved form of treatment for acute ischemic stroke. Pre-clinical research strategies include targeting anti-apoptotic and anti-inflammatory mechanisms.

The pathophysiological responses to traumatic brain injury (e.g., brain injury caused by, among other things, head accidents and head wounds) are similar in many respects to those of stroke and similar approaches are being taken to develop therapeutics for the treatment of traumatic brain injury. Whether or not a stroke is caused by ischemic or hemorrhagic mechanisms can be determined by a CAT scan or other clinical procedure and the mode of subsequent treatment will be dependent upon the results of this screening.

Spinal cord injury and trauma, like traumatic brain injury, can occur in a young healthy population but shares many pathological similarities to the changes occuring in the brain after a stroke. In light of such common mechanisms similar therapeutic approaches may be useful for treating stroke, traumatic brain injury and spinal cord injury.

Patients who have suffered a stroke or spinal cord injury, can have low blood pressure. Because of the low blood pressure, these patients may not be able to use many vasodilators or nitric oxide donors. However, inhaled nitric oxide can be used successfully with these patients.

Nitric oxide (NO) may also be a promising treatment for these patients because nitric oxide has been shown to have both cardioprotective and neuroprotective properties. The protective effects of inhaled nitric oxide may be associated with reduced water diffusion abnormality, reduced caspase-3 activation, reduced cytokine induction in the brain, and increased serum nitrate/nitrite levels. *See* Minamishima, Am. J. Physiol. Heart Circ. Physiol., April 2011, 300:H1477-H1483; doi:10.1152/ajpheart.00948, which is incorporated by reference in its entirety. These results may occur via soluble guanylate cyclase-dependent mechanisms.

One method of treating a patient can include delivering an effective concentration of the nitric oxide to the patient. Delivery of the nitric oxide can occur after the patient has experienced an ischemic or reperfusion injury, an event resulting in inflammation in the central nervous system or after inflammation resulting from a trauma to the central nervous system has been diagnosed. In some embodiments, an event resulting in inflammation in the central nervous system or a trauma to the central nervous system can include a stroke or spinal cord injury.

Nitric oxide can be delivered to a patient between at least 15 minutes, at least 30 minutes, at least 45 minutes, at least 1 hour, at least 2 hours, at least 3 hours, at least 4 hours or at least 6 hours, or at most 6 hours, at most 4 hours, at most 3 hours, at most 2 hours, at most 1.5 hours, at most 1.25 hours or at most 1 hour after an event or injury. Preferably, nitric oxide can be delivered to a patient between about 15 minutes and about 3 hours, between about 30 minutes and 2 hours or between about 45 minutes and about 1.25 hours after an event or injury.

The effective amount or effective concentration administered to treat a patient who has suffered an ischemic/reperfusion injury or an event resulting in inflammation in the central nervous system can be selected from the dosing and delivery described above. Further, any of the devices described above can be utilized for treating a patient who has suffered an ischemic/reperfusion injury or an event resulting in inflammation in the central nervous system.

Organ Failure In Liver Transplant Patients

Ischemia/reperfusion (IR) injury in transplanted livers can contribute to organ dysfunction and failure and can be characterized in part by loss of NO bioavailability. Inhaled nitric oxide may limit ischemia-reperfusion injury in transplanted livers. As a result, inhaled NO can decrease hospital length of stay, and can improve the rate at which liver function was restored after transplantation.

See Inhaled NO accelerates restoration of liver function in adults following orthotopic liver transplantation. J Clin Invest. 2007 Sep;117 (9):2583-91; Inhaled NO accelerates restoration of liver function in adults following orthotopic liver transplantation. J Clin Invest. 2007 Sep;117 (9):2583-91; Study to Evaluate if Inhaled Nitric Oxide Improves Liver Function After Transplantation NCT00582010, each of which is incorporated by reference in its entirety.

The effective amount or effective concentration administered to a patient to treat organ failure following a liver transplant can be selected from the dosing and delivery described above. Further, any of the devices described above can be utilized for treating a patient who has organ failure following a liver transplant.

Right Heart Failure/ RV Dysfunction After LVAD Insertion

NO can improve hemodynamics in patients with respiratory failure, shock, and right ventricular dysfunction. Inhaled nitric oxide may be useful in managing RV failure. Nitric oxide can decrease pulmonary vascular resistance without reducing systemic pressures. Nitric oxide may also enhance the efficacy of inotropic therapy by reducing the afterload and allowing for greater right-sided cardiac output. Implantation of a left ventricular assist device (LVAD) as a bridge to transplantation has become an acceptable approach for patients with end-stage heart failure. Right heart failure / RV dysfunction after insertion of a left ventricular assist device can occur in 20-50% of patients receiving LVAD. Nitric oxide can reduce PA pressures and can improve LVAD flow in the setting of RV dysfunction, reducing the need for mechanical RV support.

Patients undergoing LVAD placement for end-stage heart failure who manifested hemodynamically significant elevations in PVR and signs of right ventricular failure can experience reductions in PVR that were manifested as decreases in PAP and increases in LVAD-assisted cardiac output after receiving inhaled NO. Inhaled NO can be useful intraoperative adjunct in patients undergoing LVAD insertion in which pulmonary hypertension limits device filling and output. (See Right ventricular failure after left ventricular assist device insertion: preoperative risk factors, Interact CardioVasc Thorac Surg, 2006, 5:379-382; Randomized, double-blind trial of inhaled nitric oxide in LVAD recipients with pulmonary hypertension, Ann Thorac Surg., 1998, 65:340-345; ClinicalTrials.gov Identifier: NCT00060840, each of which is incorporated by reference in its entirety).

The effective amount or effective concentration administered to a patient to treat right heart failure/ RV dysfunction after LVAD insertion can be selected from the dosing and delivery described above. Further, any of the devices described above can be utilized for treating a patient who has right heart failure/ RV dysfunction after LVAD insertion.

Treatment of Cardiovascular Shock due to RV myocardial Infarction/Right Ventricular Failure (Cardiovascular Shock) Infarct (Heart Failure)

Cardiogenic shock can be the leading cause of death among patients hospitalized with acute myocardial infarction. Right ventricular myocardial infarction (RVMI) can be observed in up to 50% of patients with acute left ventricular (LV) inferior posterior wall infarction. After load reduction therapy for the failing RV with a selective pulmonary vasodilator might be expected to lead to improved cardiac performance without producing systemic vasodilation and hypotension. Nitric oxide inhalation can result in acute hemodynamic improvement when administered to patients with RVMI and CS. Patients with cardiogenic shock due to RVMI, who were administered NO (80 ppm) acutely, can demonstrate decreased mPAP and mRAP by 12% and improved cardiac index by 24%. Treatment with inhaled NO can decrease shunt flow by 56% and can be associated with markedly improved systemic oxygen saturation. (See Hemodynamic effects of inhaled nitric oxide in right ventricular myocardial infarction and cardiogenic shock, J Am Coll Cardiol, 2004, 44:793-8; The Role of Nitric Oxide and Vasopressin in Refractory Right Heart Failure Journal of Cardiovascular Pharmacology and Therapeutics, Vol. 9, No. 1, 9-11 (2004); Yoshida et al., The effect of low-dose inhalation of nitric oxide in patients with pulmonary fibrosis, Eur Respir J, (1997) 10:2051-4; Hemodynamic effects of inhaled nitric oxide in right ventricular myocardial infarction and cardiogenic shock, J Am Coll Cardiol, 2004, 44:793-8, each of which is incorporated by reference in its entirety).

The effective amount or effective concentration administered to a patient to treat cardiovascular shock due to RV myocardial infarction / right ventricular failure (cardiovascular shock) infract (heart failure) can be selected from the dosing and delivery described above. Further, any of the devices described above can be utilized for treating a patient who has cardiovascular shock due to RV myocardial infarction / right ventricular failure (cardiovascular shock) infract (heart failure).

Ischemia-Reperfusion Injury After Coronary Artery Bypass Surgery (CABG)

Significant complications associated with CABG can increase mortality and morbidity. Myocardial reperfusion that can occur during CABG can also result in significant cardiac injury. The protective actions of NO in ischemia and reperfusion can be due to its antioxidant and anti-inflammatory properties. Additionally, NO can have beneficial effects on cell signalling and inhibition of nuclear proteins, such as NF-kappa B and AP-1. NO can result in improvement in oxygenation and reduction in pulmonary arterial pressures post-operatively in CABG patients. (See 2006 national hospital discharge survey, Natl Health Stat Rep No.5, at www.cdc.gov/nchs/data/nhsr/nhsr005; Nitric oxide mechanisms of protection in ischemia and reperfusion injury, J Invest Surg, 22:46-55, each of which is incorporated by reference in its entirety).

The effective amount or effective concentration administered to a patient to treat ischemia-reperfusion injury after coronary artery bypass surgery (CABG) can be selected from the dosing and delivery described above. Further, any of the devices described above can be utilized for treating a patient who has an ischemia-reperfusion injury after coronary artery bypass surgery (CABG).

Adjunct Therapy in STEMI And Non-STEMI ACS With Planned PCI Acute Coronary Syndromes (ACS)

Reperfusion of ischemic myocardium during the treatment of MI may result in paradoxical myocardial injury compromising myocardial salvage and left ventricular functional recovery. Nitric oxide can modulate many of the processes contributing to ischemia-reperfusion injury (IR) and inhaled NO can decrease infarct size in animal models of IR. The pulmonary vasodilatory effect of inhaled NO can reduce pulmonary vascular pressure, thereby decreasing the stress on the right heart, which can be another potential benefit of inhaled NO therapy following a MI. Furthermore, inhaled NO can also have thrombolytic and anti-coagulant effects that may be useful for treating MI, as antiplatelet and anti-coagulants are a standard treatment, particularly in ACS.

The benefits of inhaled NO can extend to three key aspects of STEMI / NSTEMI management: 1) increased oxygen supply to the myocardium, enhancing oxygenation and reducing reperfusion injury and infarct size, 2) reduced stress on the heart by reducing pulmonary vascular pressure, and 3) desirable regulation of blood clotting (i.e., thrombolytic and anti-coagulant effect).

At higher doses, for example, greater than 30 ppm, greater than 40 ppm, greater than 50 ppm, greater than 60 ppm, greater than 70 ppm, greater than 80 ppm, or greater than 90 ppm, but most preferably between 40 ppm and 80 ppm, inhaled NO can alter systemic vascular resistance and response to nitric oxide synthase inhibitors in experimental models, suggesting a systemic vascular effect. Moreover, inhaled NO can reduce infarct size in rodent models of myocardial infarction.

Anecdotal experience indicates a beneficial impact of inhaled NO on the hemodynamic course of patients with right ventricular MI and warrants further investigation. There are ongoing randomized placebo-controlled clinical trials of inhaled NO in STEMI treated by primary angioplasty (See ClinicalTrials.gov Identifier: NCT00854711, which is incorporated by reference in its entirety) and in reduction of Myocardial Infarction Size (SeeClinicalTrials.gov Identifier: NCT00568061, which is incorporated by reference in its entirety). (See also Hemodynamic effects of inhaled nitric oxide in right ventricular myocardial infarction and cardiogenic shock,J Am Coll Cardiol, 44:793-798; Inhaled NO as a Therapeutic Agent, Cardiovascular Research, 2007 May 7, 75:339-48; ClinicalTrials.gov Identifier: NCT00854711; ClinicalTrials.gov Identifier: NCT00568061, each of which is incorporated by reference in its entirety).

The effective amount or effective concentration administered to a patient to treat STEMI or NON-STEMI ACS with planned PCI acute coronary syndromes (ACS) can be selected from the dosing and delivery described above. Further, any of the devices described above can be utilized for treating a patient who has STEMI or NON-STEMI ACS with planned PCI acute coronary syndromes (ACS).

Traumatic Brain Injury (TBI)

Nitric oxide can improve the outcome for a patient who has suffered from an ischemic (or hypoxic) injury and/or a reperfusion injury. Examples of these types of injuries can include injuries resulting from cardiac arrest, stroke, aneurism, strangulation, suffocation, hypothermia, respiratory trauma, and central nervous system trauma, including spinal cord trauma.

Central nervous system ("CNS") injuries and trauma encompass a wide variety of medical and traumatic insults to the brain and spinal cord. For example, stroke is the third leading cause of death in the developed world with one stroke occurring approximately every minute in the United States. Mortality rate is about 30% but more than 4 million stroke survivors are alive today, the majority of these individuals are left with varying degrees of disability. Clinical trials have yet to demonstrate therapeutic neuroprotection in ischemic stroke (i.e., stroke related to disruption of blood flow due to clot/thrombus formation) and spinal cord. Thrombolytic therapy (defined as use of an agent which causes dissolution or destruction of a thrombus) has many limitations, but it remains the only approved form of treatment for acute ischemic stroke. Pre-clinical research strategies include targeting anti-apoptotic and anti-inflammatory mechanisms.

The pathophysiological responses to traumatic brain injury (e.g., brain injury caused by, among other things, head accidents and head wounds) are similar in many respects to those of stroke and similar approaches are being taken to develop therapeutics for the treatment of traumatic brain injury. Whether or not a stroke is caused by ischemic or hemorrhagic mechanisms can be determined by a CAT scan or other clinical procedure and the mode of subsequent treatment will be dependent upon the results of this screening.

Spinal cord injury and trauma, like traumatic brain injury, can occur in a young healthy population but shares many pathological similarities to the changes occuring in the brain after a stroke. In light of such common mechanisms similar therapeutic approaches may be useful for treating stroke, traumatic brain injury and spinal cord injury.

For example, inhaled nitric oxide may decrease the inflammatory response in patients with increased intracranial pressure caused by traumatic brain injury accompanied by acute respiratory distress syndrome, thereby contributing to improved outcomes. No adverse cerebral effects with NO therapy in a child with traumatic brain injury have been observed. (See The Influence of Inhaled Nitric Oxide on Cerebral Blood Flow and Metabolism in a Child with Traumatic Brain Injury, Anesth Analg, 2001, 93:351-3; The beneficial effects of inhaled nitric oxide in patients with severe traumatic brain injury complicated by acute respiratory distress syndrome: a hypothesis, Journal of Trauma Management & Outcomes, 2008, 2:1; Successful Use Of Inhaled Nitric Oxide To Decrease Intracranial Pressure In A Patient With Severe Traumatic Brain Injury Complicated By Acute Respiratory Distress Syndrome: A Role For An Anti-Inflammatory Mechanism? Scandinavian Journal of Trauma, Resuscitation and Emergency Medicine, 2009, 17:5, each of which is incorporated by reference in its entirety.

The effective amount or effective concentration administered to a patient to treat TBI can be selected from the dosing and delivery described above. Further, any of the devices described above can be utilized for treating a patient who has TBI.

Pulmonic Valve Insufficiency / Pulmonary Valve Regurgitation (PI) In TOF / CHD

Pulmonic valve insufficiency (PI) can be a problem after primary surgical repair of Tetralogy of Fallot (TOF). TOF can be a congenital heart defect. Long-term PI can lead to structural changes in the right ventricle, the sequelae of which include right heart failure, arrhythmia, and sudden cardiac death. The only current treatment for severe symptomatic PI is pulmonic valve replacement. Inhaled nitric oxide can have acute effects on pulmonary insufficiency in congenital heart disease. (See ClinicalTrials.gov Identifier NCT00543933; ClinicalTrials.gov Identifier NCT00543933, each of which is incorporated by reference in its entirety.)

The effective amount or effective concentration administered to a patient to treat pulmonic valve insufficiency/ pulmonary valve regurgitation (PI) in TOF/CHD can be selected from the dosing and delivery described above. Further, any of the devices described above can be utilized for treating a patient who has pulmonic valve insufficiency/ pulmonary valve regurgitation (PI) in TOF/CHD.

Pulmonary Embolism

Acute pulmonary embolism can increase pulmonary vascular resistance by reduction of the cross sectional area of the pulmonary vascular bed and also by vasoconstriction. Inhaled NO has the potential to reduce right ventricular after load both by vasodilation and by its impact on platelet aggregation. Inhaled NO produced improvements in systemic blood pressures, heart rate and gas exchange in patents suffering massive pulmonary embolism. (See Inhaled Nitric Oxide Improves Pulmonary Functions Following Massive Pulmonary Embolism: A Report of Four Patients and Review of the Literature, Lung, 2006, 184:1-5, each of which is incorporated by reference in its entirety.)

The effective amount or effective concentration administered to a patient to treat pulmonary embolism can be selected from the dosing and delivery described above. Further, any of the devices described above can be utilized for treating a patient who has a pulmonary embolism.

Cystic Fibrosis (CF)

NO can be important in host defense due to its antibacterial properties. In the setting of CF, a correlation had been demonstrated between low airway NO levels and chronic airway colonization with *Pseudomonas aureginosa.* Bacterial colonization can result from low airway NO levels in CF infants. Therefore, inhaled NO may have protective properties with regard to pulmonary bacterial infection. (See ClinicalTrials.gov Identifier NCT00570349; Airway Nitric Oxide in Patients With Cystic Fibrosis Is Associated With Pancreatic Function, Pseudomonas Infection, and Polyunsaturated Fatty Acids, Chest 2007, 131:1857-1864, each of which is incorporated by reference in its entirety.)

CF patients are generally children. Children can include patients who are less than 13 years old, less than 10 years old or less than 5 years old.

However, CF patients can also include adolescents. Adolescents can include patients who are between the ages of 13 and 18 years old.

A patient with CF can be a newborn. A newborn can be patient who is less than 1 year old, less than 6 months old, less than 3 months old, less than 2 months old or less than 1 month old. Most preferably, a newborn is less than 3 months old. A newborn may or may not have been born after full gestation (e.g. 40 weeks gestation). For example, a newborn could have been born premature, for example, having a gestation period of less than 40 weeks, more specifically, less than 36 weeks. An infant can be a patient who is less than 1 year old.

The effective amount or effective concentration administered to a patient to treat CF can be selected from the dosing and delivery described above. Further, any of the devices described above can be utilized for treating a patient who has CF.

Sepsis (Augment Tissue Perfusion In Sepsis)

Microcirculatory dysfunction can be an element in the pathogenesis of sepsis and can cause impairment of tissue perfusion independent of global hemodynamics. The highest indices of microcirculatory dysfunction can be found among non-survivors with sepsis. NO can protect microcirculatory patterns. Exogenous NO may be able to preserve microcirculatory flow in sepsis, thereby opening low flow microcirculatory units via the modulation of microvascular tone and reducing the adhesiveness of microvascular endothelium. (See Clinicaltrials.gov identifier NCT00608322; Airway Nitric Oxide in Patients With Cystic Fibrosis Is Associated With Pancreatic Function, Pseudomonas Infection, and Polyunsaturated Fatty Acids, Chest 2007, 131:1857-1864, each of which is incorporated by reference in its entirety.)

The effective amount or effective concentration administered to a patient to treat sepsis can be selected from the dosing and delivery described above. Further, any of the devices described above can be utilized for treating a patient who has sepsis.

Cerebral malaria

Approximately 200 million cases of cerebral malaria develop annually and approximately 1 million people die each year of cerebral malaria. Approximately 98% of those deaths occur in the developing world, where access to hospitals or other treatment facilities can be limited. Further, approximately twenty-five percent of children who survive cerebral malaria are left with long-term brain injury and disabilities. (Black, D., "Has malaria met its match?", Toronto Star, October 10, 2011).

Severe malaria decreases the production of nitric oxide in the body, leading to harmful effects. Administration of nitric oxide to mice models of malaria have shown an increased chance of survival and protection by nitric oxide against neurological damage.

Therefore, it has been suggested that NO may improve the survival rates of patients with cerebral malaria. Treatment of these patients in remote and poverty stricken parts of the world may only be possible by means of device which is capable of working in such locations, such as the delivery devices described herein.

Cerebral malaria patients can be children. Children can include patients who are less than 13 years old, less than 10 years old or, for cerebral malaria, most commonly, less than 5 years old.

However, cerebral malaria patients can also include adolescents or adults. Adolescents can include patients who are between the ages of 13 and 18 years old. Adults can be ages 18 and older.

The effective amount or effective concentration administered to a patient to treat cerebral malaria can be selected from the dosing and delivery described above. Further, any of the devices described above can be utilized for treating a patient who has cerebral malaria.

Battlefield lung injury

Nitric oxide is a selective pulmonary vasodilator that improves pulmonary perfusion and ventilation-perfusion matching. Due to the physiological effects of NO, delivery of ultra-pure NO with air soon after injury may stabilize wounded fighters with thoracic injuries more quickly and reduce the risks of evacuation (transport) better than oxygen therapy, thereby increasing chances for survival. Prior limited studies (Papadimos, 2009) report positive outcomes (increased PaO2, survival) for conventional iNO treatment of traumatic brain injury (TBI) in patients with acute lung injury/acute respiratory distress syndrome (ALI/ARDS), indicating that additional focused human testing of iNO efficacy is warranted.

While conventional NO is already FDA-approved for use in treating respiratory disease in neonates (Ikaria's INOmax®), the technique for generating and delivery NO has two major drawbacks for military applications: 1) this method requires the transport of large, pressurized gas tanks with complex monitors, which is both impractical and unsafe on the battlefield; and 2) this method necessarily co-delivers toxic levels of $NO_2$.

With conventional methods, pressurized NO gas in $N_2$ (800 ppm NO) is mixed with oxygen or air prior to inhalation. As $NO_2$ formation is proportional to the square power of source NO concentration, this approach results in significant $NO_2$ formation. For example, with an inhaled NO dose of 80 ppm, as much as 3 ppm of $NO_2$ can be co-delivered when using the conventional technique. The National Institute for Occupational Safety and Health (NIOSH) limit is 1 ppm for a healthy worker for 15 minutes, and the proposed limit from the Environmental Protection Agency (EPA) is 0.1 ppm. It has been suggested that previous studies of NO efficacy using conventional medical-grade NO for treating respiratory disease/injury (including ALI/ARDS) may have been impacted by $NO_2$ toxicity (Lowson, 2005), which may have limited the outcomes.

Fourteen-day toxicity studies using the delivery devices described above with rats and dogs have successfully been completed at doses well in excess of 80 ppm NO.

**[0290]** As the delivery devices described above are simpler and portable, the devices can be used to stabilize patients and allow patients to tolerate longer evacuation times, which can improve patient outcomes and decreasing mortality. The devices may eliminate or substantially reduce the amount of supplemental $O_2$ needed for rescues at all altitudes. Therefore, the devices described above may assist in aiding soldier or other military personnel at sites of combat. For example, a one lb deliver device as described can be more practical to use by pararescue medics than an eighteen lb portable $O_2$ system, which can increase the quality of combat care. Consequently, any of the devices described above can be utilized for treating a patient who has a battlefield injury.

The effective amount or effective concentration administered to a patient to treat battle-field injuries can be selected from the dosing and delivery described above.

Persistent Pulmonary Hypertension Of The Newborn

FDA has approved the use of inhaled NO for the treatment of newborns with hypoxic respiratory failure associated with clinical or echocardiographic evidence. More than 294,000 patients have been treated with Ikaria's INOmax® in the US alone since its approval for PPHN in 1999. Clinical trials have demonstrated that inhaled NO safely improves arterial oxygen levels and decreases the need for Extracorporeal Membrane Oxygenation (ECMO). However, the current method is limited by the use of gas bottles.

A newborn can be patient who is less than 1 year old, less than 6 months old, less than 3 months old, less than 2 months old or less than 1 month old. Most preferably, a newborn is less than 3 months old. A newborn may or may not have been born after full gestation (e.g. 40 weeks gestation). For example, a newborn could have been born premature, for example, having a gestation period of less than 40 weeks, more specifically, less than 36 weeks. An infant can be a patient who is less than 1 year old.

Pulmonary hypertension has been described in greater detail above. Briefly, however, persistent pulmonary hypertension of the newborn can be a condition that results when the ductus arteriosus remains open and a newborn's blood flow continues to bypass the lungs. As a result, oxygen does not reach the bloodstream, even though the baby is breathing. Persistent pulmonary hypertension of the newborn can be either a primary condition or secondary condition (i.e. results from another condition or disorder).

Use of a device as described herein can simplify the delivery procedure and can allow for a dose in constant milligrams, as compared to constant ppm. The liquid source can also achieve a constant dose within a breath that can vary by less than 1%, less than 2%, less than 5% or less than 10% of the mean. This is an improvement compared to what FDA allows today, which is a variation of from 0 to 150% of the mean.

The effective amount or effective concentration administered to a patient to treat persistent pulmonary hypertension of the newborn can be selected from the dosing and delivery described above.

HIGH ALTITUDE ILLNESS (HAI) AND ACUTE MOUNTAIN SICKNESS/HIGH ALTITUDE PULMONARY EDEMA (HAPE)

Exposure to low oxygen environments, typically found in high altitudes, may cause an individual to develop high-altitude sickness. The high altitude can be an altitude greater than 8,000 feet above sea level, greater than 10,000 feet above sea level, greater than 12,000 feet above sea level, greater than 14,000 feet above sea level, greater than 16,000 feet above sea level, greater than 18,000 feet above sea level, and higher.

High-altitude sickness can be relatively mild to life-threatening. A relatively mild form of high altitude sickness is acute mountain sickness, which is characterized by symptoms such as, but not limited to, headaches, breathlessness, fatigue, nausea, vomiting, or sleeplessness. Life-threatening forms of high-altitude sickness include high-altitude pulmonary edema (HAPE) and high-altitude cerebral edema (HACE). HAPE is characterized by symptoms such as pulmonary hypertension, increased pulmonary capillary permeability, and hypoxemia. HACE is characterized by changes in behavior, lethargy, confusion, and loss of coordination.

Elevated pulmonary artery pressure (PAP) caused by hypoxic pulmonary vasoconstriction (HPV) can be a key prerequisite for the development of high altitude primary edema (HAPE) and thus the reduction of PAP can be important in HAPE prophylaxis and treatment. At high altitude, inhaled NO can cause a significantly greater reduction in the systolic PAP of HAPE-susceptible individuals compared to its effect on the PAP of HAPE-resistant subject.

Typically, a mild case of high-altitude sickness is treated with rest, fluids, analgesics, or dexamethasone. More severe cases of high-altitude sickness can be treated with oxygen, hyperbaric therapy, or descent to lower elevations. While oxygen and hyperbaric therapies and descent to lower elevations provide relief from high-altitude sickness, these treatments have shortcomings. For example, oxygen therapy requires heavy, gas bottles that are difficult to carry in higher elevations. Hyperbaric therapy is less than ideal because this treatment requires specialized equipment and is labor-intensive. Lastly, descent to lower elevations may be not possible due to environmental factors or the poor physical condition of the individual. Accordingly, there remains a need for treatments of high-altitude sickness.

Inhaled NO can improve arterial oxygenation and diminished pulmonary arterial pressure in patients with profound hypoxemia, moderately severe pulmonary hypertension and overtly symptomatic pulmonary edema. (See, for example, Treatment of Acute Mountain Sickness and High Altitude Pulmonary Edema, MJAFI 2004, 60:384-38; Randomized, Controlled Trial of Regular Sildenafil Citrate in the Prevention of Altitude Illness NCT00627965; Inhaled NO and high altitude, N. Engl. J. Med., 1996, 334:624-9, Effects of Inhaled Nitric Oxide and Oxygen in High-Altitude Pulmonary Edema, Circulation, 1998, 98:2441-2445; Treatment of Acute Mountain Sickness and High Altitude Pulmonary Oedema MJAFI, 2004, 60:384-387; Acute Mountain Sickness, High Altitude Cerebral Oedema, High Altitude Pulmonary edema: The Current Concepts, MJAFI, 2008, 64:149-153, each of which is incorporated by reference in its entirety.)

The use of inhaled NO and oxygen together can cause an additive effect on pulmonary hemodynamics and gas exchange. (See High-altitude pulmonary oedema: still a place for controversy?, Thorax, 1995, 50:923-929, which is incorporated by reference in its entirety.) Additionally, NO can significantly improve the outcome of HAPE cases when compared to a control group. *Id.* NO and oxygen were found to be working through different ion channels, which thereby increased synergy in treatment.

The problem has been that treatment of HAI and HAPE with NO can require extensive equipment, including a gas bottle containing a mixture of NO in nitrogen at high pressure, a pressurized oxygen gas bottle, gas regulators to control the flow out of the pressurized gas bottles, mixing hardware, chemical monitoring instruments for NO, oxygen and $NO_2$. This level of equipment can only be made portable with a large vehicle. Therefore, it can be unusable for prevention and for treatment when evacuation is not possible.

Using the devices described above, it may not only be possible to treat patients that have HAI and HAPE, but it can be used as a prophylactic to prevent the occurrence in the first place. An advantage in the ability to prevent and treat HAPE and HAI can be the portable nature of the disclosed portable devices. These devices can make it possible to use the device for people at high altitudes, including mountain climbers, tourists who visit communities at high altitude, helicopter pilots or soldiers who need to work and fight at high altitude.

According to one method, a therapeutic amount of nitric oxide (NO) is delivered to an individual's lungs when the individual is at high altitude. The delivery can take place before, during or after the onset of symptoms of high-altitude sickness. NO is inhaled continuously or intermittently for a few minutes to one or more days. In another method, air, oxygen-enriched air, or substantially pure oxygen may also be delivered with NO to treat high-altitude sickness.

The treatment of HAPE or HAI or other forms of high-altitude sickness have been described in detail, for example, in U.S. Patent No. 2010/0043788, which is incorporated by reference in its entirety.

The effective amount or effective concentration administered to a patient to treat high-altitude sickness, HAPE or HAI can be selected from the dosing and delivery described above.

WOUND HEALING

Nitric oxide has antibacterial, antiviral, and antifungal properties. See, for example, US Patent Nos. 7,520,866, 7,192,018 and 6,793,644, each of which is incorporated by reference. As microorganisms are not immune to NO, NO is potentially effective against all bacteria, viruses, fungi and parasites.

A wound can be an injury to the body. A wound can include a laceration or breaking of a membrane, such as the skin. A wound also can include damage to underlying tissues.

Wounds can occur in many places on the body. One common location can be on a foot. For example, while diabetic sores can occur in many places, one frequent location of diabetic sores can be on a foot. Limited clinical trials using NO to treat infections that are common with diabetic sores have been undertaken and have demonstrated favorable results. Additionally, NO treatment can also be effective against fungal infections of the feet, such as Athlete's Foot.

While any of the devices described above can be utilized, a special bandage can also be connected to one of the devices or another source of NO to treat wounds. In particular, a bandage can be a boot (for the foot), a glove (for the hand) or any other specialized, close-fitting covering that can contact a wound.

Unlike applications which involve inhaling nitric oxide, doses administered to wounds can be relatively high, for example, at least about 50 ppm, at least about 75 ppm, at least about 100 ppm, at least about 125 ppm, at least about 150 ppm, at least about 175 ppm, or at least about 200 ppm. The need to administer such a high dose may be due to the administration in a medical facility, where NO is typically stored in gas cylinders that are not practical for home use. Home use can be dangerous, especially when the concentration may be high enough to be hazardous. The dose of nitric oxide in the doctor's office can be followed by several hours at a lower dose on subsequent days.

A liquid source can change this utility dynamic. A liquid source of NO can be made part of a bandage and can eliminate need for gas bottles. A relatively small source permeating a low level of nitric oxide can allow for home use and the treatment can be more widely used.

A simple bandage can be used that has an outer layer. The outer layer can slow the permeation rate of air, similar to having a lining of vinyl or other plastic. The liquid source can have a seal. Once the bandage is in place and the seal to the NO is broken, the NO can be released to the wound or sore. The patient's own body heat can be adequate to convert the liquid $NO_2$ to gaseous NO, especially because the temperature need not be exact since the precise dose may not be important. The effective amount or effective concentration administered to a patient to treat a wound can be selected from the dosing and delivery described above. In some embodiments, a dose can be at least about 1 ppm, at least about 2 ppm, at least about 5 ppm, at least about 8 ppm, at least about 10 ppm, at least about 12 ppm, at least about 15 ppm, at least about 18 ppm or at least 20 ppm of nitric oxide. Preferably, the dose of nitric oxide can be in the range between about 1 and about 50 ppm, in the range between about 1 and about 30 ppm, or most preferably in the range of about 5 to about 20 ppm. The device could include either a permeation tube or a diffusion cell, as both would work well.

One method can be, for example, for treating a wound or infection that was not healing. The method can include placing an NO bandage on the wound and leaving the bandage in position for a period of time, for example, at least 1 hour, at least 2 hours, at least 3 hours, at least 5 hours, at least 6 hours, at least 12 hours, at least 24 hours, at least two days or more. The method could include destroying or inhibiting the growth of any microorganisms growing or present in the wound. This could allow the wound to heal. This method could eliminate a need to come to the doctor's office and could allow the treatment to be performed by the patient.

A method can further include heating the NO with small heaters to administer an initial high dose. An initial high dose can be at least about 50 ppm, at least about 75 ppm, at least about 100 ppm, at least about 125 ppm, at least about 150 ppm, at least about 175 ppm, or at least about 200 ppm, as discussed above.

In some circumstances, the method can further include allowing the temperature to fall to body temperature, thereby administering a long sustained dose.

An initial temperature spike could require a heating device, such as a heater. In a preferred embodiment, a heating source can be commercially available chemical heaters, for example, heaters that provide warmth to the feet while watching a ball game in the winter. A commercially available chemical heater can include a pouch with water and a pouch with a chemical, for example soda lime, which are allowed to mix, generating the heat of solution. In another preferred embodiment, a heater can be a battery type of heater.

EXAMPLES:

EXAMPLE 1

The table below was generated with an air flow of 1 LPM air (using a mass flow controller), with an ascorbic acid/silica gel powder ribbed reactor. The $NO_2$ was supplied from a reservoir heated to 61°C in a water bath. The NO reading is approximately 79 ppm. The fused quartz tube was 25 micron id and supplied by Restek as a "Guard column" ("GC"). The length of the GC column started at 39.88 inches. The GC column (except the last 2 inches) and liquid vessel are submerged in the water bath. Table 3 shows the relationship between length and concentration from this experiment.

**TABLE 3**

| Length [inches] | Concentration NO [ppm] | Calculated Concentration NO [ppm] | % Off | GC Tubing Removed [inches] | Temperature [C] | Set Flow rate [LPM] |
|---|---|---|---|---|---|---|
| 88.00 | 36.80 | NA | NA | | 61.8 | 1 |
| 76.50 | 41.95 | 42.33 | -0.91% | 11.5 | 621 | 1 |
| 64.25 | 50.33 | 50.40 | -0.14% | 12.25 | 61.4 | 1 |
| 50.00 | 63.80 | 64.77 | -1.52% | 14.25 | 61 | 1 |
| 39.88 | 79.00 | 81.21 | -2.80% | 10.125 | 61.3 | 1 |

The results show that within the limits of experimental error the output is inversely proportional to the length.

EXAMPLE 2

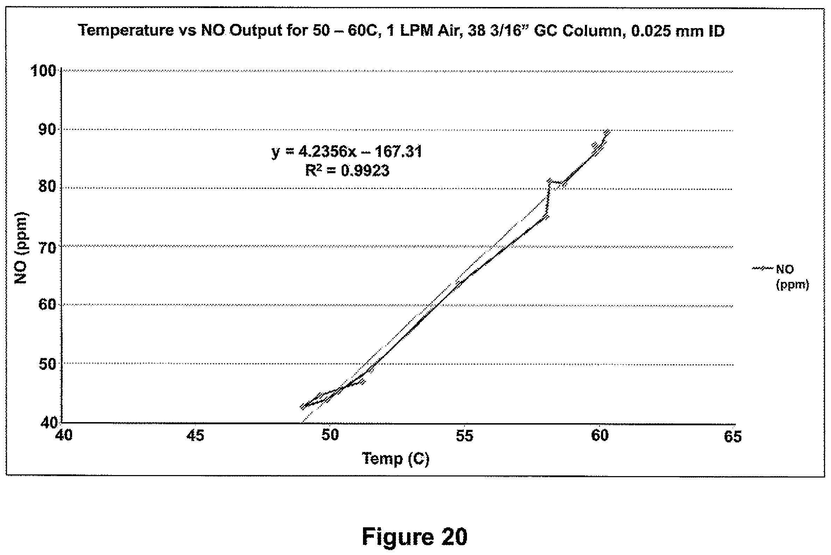

In this example, the length of the 25 micron diameter tube was held at 38 3/16 inches. The cartridge can be a ribbed tube that was packed with the ascorbic acid/silica gel powder. The temperature of the storage vessel and the tube were varied from about 49°C to just over 60°C. FIG. 20 demonstrates that over this temperature range, the increase in output was approximately linear, increasing 10-fold from 44 ppm at 50°C to 88 ppm at 60°C.

EXAMPLE 3

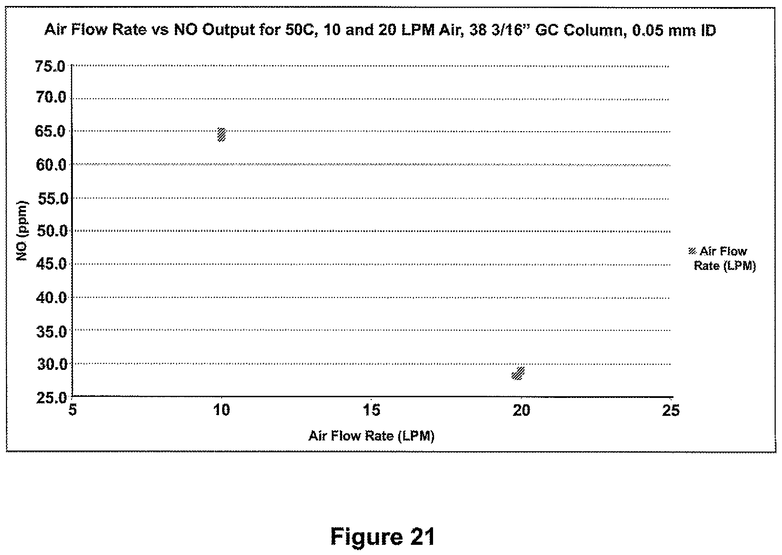

In this example a tube with a 50 micron id tube was used. The output of this tube was 64 ppm at 10 liter per minute and 28 ppm at 20 liters per minute; doubling the flow of air resulted in the output being halved, as expected. See FIG. 21. For this diameter, the expected output should vary with the $4^{th}$ power of the diameter as compared to a tube of 25 microns, or a factor of 16. From example 2, the output at 50°C and 11 per minute was 44 ppm, which translates to an expected output of 70 ppm. This compares to the measured output of 65 ppm, which is within the limits of experimental error.

EXAMPLE 4

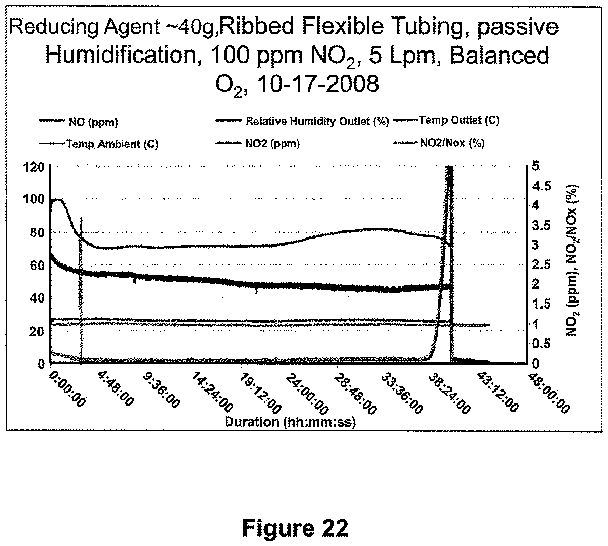

In this example, a ribbed flexible tubing was used. The rubbed tube was packed with 40g of ascorbic acid/silica gel powder. 100ppm of $NO_2$ was supplied in oxygen at 5 Lpm. The experiment was carried out over the course of approximately 42 hours as depicted in FIG. 22. FIG. 22 further illustrates that NO was released steadily for about 40 hours.

EXAMPLE 5

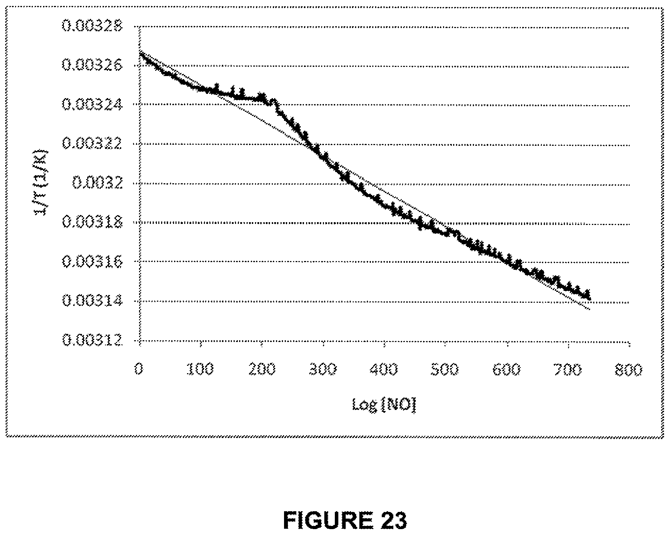

The slope of the plot of log (NO) versus 1/T, where T is the absolute temperature, should be a straight line. A typical plot obtained using a nitric oxide delivery system is shown in FIG. 23. The small variation from linearity may due to experimental error due primarily to inadequate temperature control. The flow rate was 1 liter per minute of air.

EXAMPLE 6

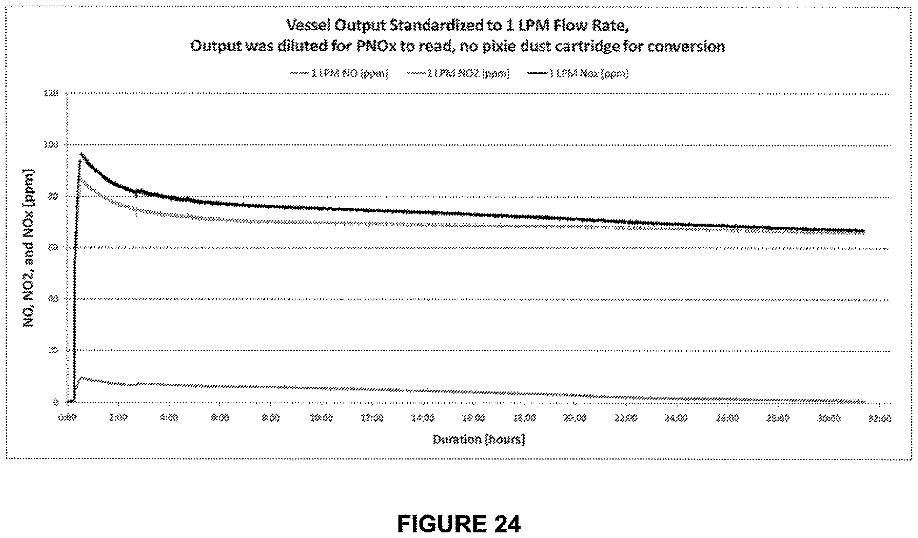

The nitric oxide delivery systems can be operated for many days on end without significant variation or degradation. For example, a typical plot of ppm NO, $NO_2$ and NO + $NO_2$ versus time is shown in FIG. 24 for one experiment over a period of about 36 hours. In this experiment the $NO_2$ to NO conversion cartridge was absent. It shows the output of the reservoir, showing the NO level (green line), the $NO_2$ level (yellow line) and the NO + $NO_2$ response (black line) with time in minutes. Without being held to any theory, the initial spike was likely due to the approximately 1% NO impurity that is sometimes added to $N_2O_4$ to reduce corrosion cracking during its conventional use as a rocket fuel oxidiser. Because it has a higher vapor pressure, the NO will de-gas from the liquid in the early stages oxidiser.

EXAMPLE 7

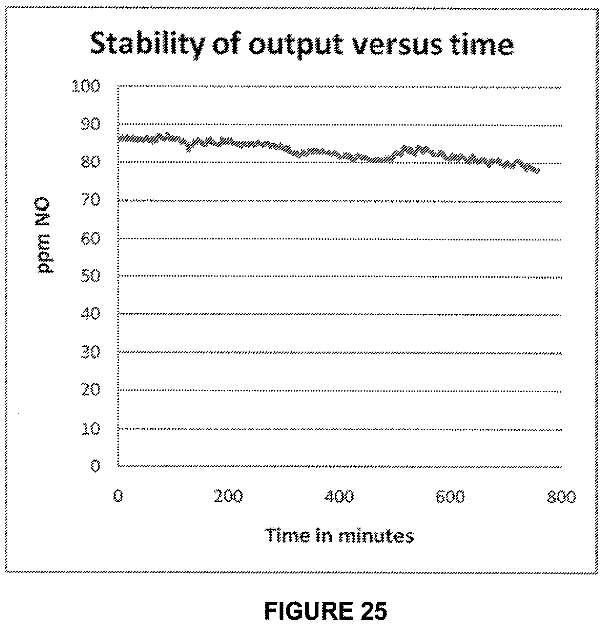

FIG. 25 shows the output when the NO conversion cartridges were included in the system to convert the $NO_2$. In this experiment, the data was collected for 780 minutes (13 hours). While the data shows some drift, it was well within the $\pm$ 20% that is required for clinical use.

EXAMPLE 8

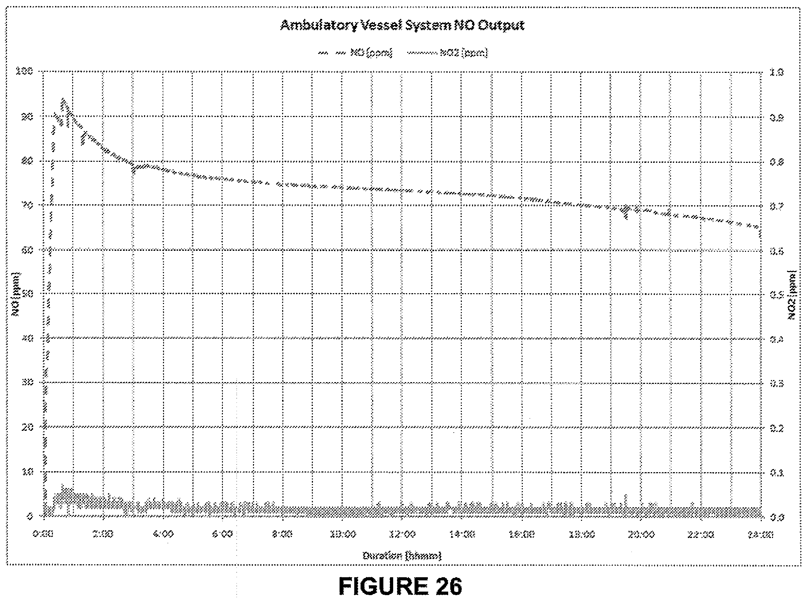

FIG. 26 shows the NO and $NO_2$ output for a period of 24 hours. The $NO_2$ concentration after the gas flow was passed through the cartridges was essentially zero.

The various embodiments described above are provided by way of illustration only and should not be construed to limit the paraed invention. Those skilled in the art will readily recognize various modifications and changes that may be made to the paraed invention without following the example embodiments and applications illustrated and described herein, and without departing from the true spirit and scope of the paraed invention, which is set forth in the following paras. Various preferred features and embodiments of the present invention will now be described with reference to the following numbered paragraphs (paras).

1. A method of treating a patient after cardiopulmonary resuscitation has been performed on the patient comprising:

passing nitrogen dioxide through a system configured to convert the nitrogen dioxide into nitric oxide; and delivering an effective concentration of the nitric oxide to the patient.

2. The method of para 1, wherein the system includes a cartridge including a surface-activated material and a reducing agent.

3. The method of para 2, wherein the reducing agent includes an antioxidant.

4. The method of para 3, wherein the antioxidant is ascorbic acid, alpha tocopherol, or gamma tocopherol.

5. The method of any one of paras 2-4, wherein the surface-activated material includes a silica gel.

6. The method of any one of paras 1-5, wherein nitric oxide is delivered to a patient between about 15 minutes and about 3 hours after cardiopulmonary resuscitation has been performed on the patient.

7. The method of any one of paras 1-6, wherein nitric oxide is delivered to a patient between about 45 minutes and about 1.25 hours after cardiopulmonary resuscitation has been performed on the patient.

8. The method of any one of paras 1-7, wherein nitric oxide is delivered to the patient for a period between 15 minutes and 48 hours.

9. The method of any one of paras 1-9, wherein nitric oxide is delivered to the patient for a period between 12 hours and 36 hours.

10. The method of any one of paras 8-9, wherein the nitric oxide is delivered to a patient continuously.

11. The method of any one of paras 8-9, wherein the nitric oxide is delivered to a patient intermittently.

12. The method of any one of paras 1-11, further comprising releasing nitrogen dioxide from a nitrogen dioxide source.

13. The method of para 12, wherein the nitrogen dioxide source is coupled to the cartridge.

14. The method of any one of paras 12-13, wherein the nitrogen dioxide source includes a nitrogen dioxide gas bottle.

15. The method of any one of paras 12-13, wherein the nitrogen dioxide source includes a reservoir including liquid dinitrogen tetroxide.

16. The method of any one of paras 1-15, wherein the cartridge includes a plurality of cartridges.

17. A method of treating a patient after the patient has experienced an event resulting in inflammation in the central nervous system comprising:

   passing nitrogen dioxide through a system configured to convert the nitrogen dioxide into nitric oxide; and
   delivering an effective concentration of the nitric oxide to the patient.

18. The method of para 17, wherein the event resulting in inflammation in the central nervous system includes a stroke or spinal cord injury.

19. The method of para 17, wherein the system includes a cartridge including a surface-activated material and a reducing agent.

20. A method of treating a patient having sleep apnea comprising:

   passing nitrogen dioxide through a system configured to convert the nitrogen dioxide into nitric oxide; and
   delivering an effective concentration of the nitric oxide to the patient.

21. The method of para 20, wherein delivering nitric oxide includes supplying forced air into the patient.

22. A method of treating a patient having pulmonary arterial hypertension comprising:

   passing nitrogen dioxide through a system configured to convert the nitrogen dioxide into nitric oxide; and
   delivering an effective concentration of the nitric oxide to the patient.

23. The method of para 22, further comprising delivering a PDE5 inhibitor to the patient.

24. A method of treating a patient having a pulmonary disorder comprising:

passing nitrogen dioxide through a system configured to convert the nitrogen dioxide into nitric oxide; and delivering an effective concentration of the nitric oxide to the patient.

25. The method of para 24, wherein the pulmonary disorder is pulmonary hypertension, chronic obstructive pulmonary disease, idiopathic pulmonary fibrosis, acute chest syndrome, infectious lung disease, hypoxemia, respiratory failure, respiratory distress syndrome, pulmonary embolism, cystic fibrosis, or combinations thereof.

26. A method of treating a patient having a cardiac or blood disorder comprising:

passing nitrogen dioxide through a system configured to convert the nitrogen dioxide into nitric oxide; and delivering an effective concentration of the nitric oxide to the patient.

27. The method of para 26, wherein the blood disorder is sickle cell.

28. The method of para 26, wherein the cardiac disorder includes heart failure or cardiovascular shock.

29. A method of treating a patient having a kidney disorder comprising:

passing nitrogen dioxide through a system configured to convert the nitrogen dioxide into nitric oxide; and delivering an effective concentration of the nitric oxide to the patient.

30. The method of para 29, wherein the kidney disorder includes alpha-1-adrenoreceptor in vasoreactivity.

31. A method of treating a patient who is attempting to quit smoking comprising:

passing nitrogen dioxide through a system configured to convert the nitrogen dioxide into nitric oxide; and delivering an effective concentration of the nitric oxide to the patient.

32. A method of treating a patient having a neurologic disorder comprising:

passing nitrogen dioxide through a system configured to convert the nitrogen dioxide into nitric oxide; and delivering an effective concentration of the nitric oxide to the patient.

33. A method of treating a patient comprising:

passing nitrogen dioxide through a system configured to convert the nitrogen dioxide into nitric oxide; and delivering an effective concentration of the nitric oxide to the patient.

34. The method of para 33, wherein the patient is postoperative.

35. The method of para 33, wherein the postoperative patient experienced pulmonary or cardiac stress.

36. The method of para 33, wherein the patient experienced ischemic injury or reperfusion injury.

37. The method of para 33, wherein the patient experienced organ failure.

38. The method of para 33, wherein the patient has altitude illness or pulmonary adema.

39. A method of treating a patient having a wound comprising:

passing nitrogen dioxide through a system configured to convert the nitrogen dioxide into nitric oxide; and exposing the wound to an effective concentration of the nitric oxide.

**Claims**

1. Nitric oxide for use in treating a patient having a pulmonary disorder, the use comprising:

   passing nitrogen dioxide through a system configured to convert the nitrogen dioxide into nitric oxide; and delivering an effective concentration of the nitric oxide to the patient.

2. The nitric oxide of claim 1 for the use of claim 1, wherein the pulmonary disorder is pulmonary hypertension, chronic obstructive pulmonary disease, idiopathic pulmonary fibrosis, acute chest syndrome, infectious lung disease, hypoxemia, respiratory failure, respiratory distress syndrome, pulmonary embolism, cystic fibrosis, or combinations thereof.

3. The nitric oxide of claim 1 or claim 2 for the use of claim 1 or claim 2, wherein the system includes a cartridge including a surface-activated material and a reducing agent.

4. The nitric oxide of claim 3 for the use of claim 3, wherein the reducing agent includes an antioxidant.

5. The nitric oxide of claim 4 for the use of claim 4, wherein the antioxidant is ascorbic acid, alpha tocopherol, or gamma tocopherol.

6. The nitric oxide of any one of claims 3-5 for the use of any one of claims 3-5, wherein the surface-activated material includes a silica gel.

7. The nitric oxide of any one of the preceding claims for the use of any one of the preceding claims, wherein the use further comprises releasing nitrogen dioxide from a nitrogen dioxide source.

8. The nitric oxide of claim 7 for the use of claim 7, wherein the nitrogen dioxide source is coupled to the cartridge.

9. The nitric oxide of any one of claims 7-8 for the use of any one of claims 7-8, wherein the nitrogen dioxide source includes a nitrogen dioxide gas bottle.

10. The nitric oxide of any one of claims 7-8 for the use of any one of claims 7-8, wherein the nitrogen dioxide source includes a reservoir including liquid dinitrogen tetroxide.

11. The nitric oxide of any one of claims 1-10 for the use of any one of claims 1-10, wherein the cartridge includes a plurality of cartridges.

Figure 1

**Figure 2**

EP 3 360 557 A2

Figure 3

EP 3 360 557 A2

EP 3 360 557 A2

**Figure 4**

Figure 5

**Figure 6**

Figure 7

EP 3 360 557 A2

Figure 8

FIGURE 9

FIGURE 10

FIGURE 11

FIGURE 12

FIGURE 13

FIGURE 14

**FIGURE 15**

EP 3 360 557 A2

**FIGURE 16**

FIGURE 17

54

FIGURE 18

FIGURE 19

Figure 20

Figure 21

Reducing Agent ~40g,Ribbed Flexible Tubing, passive Humidification, 100 ppm NO₂, 5 Lpm, Balanced O₂, 10-17-2008

Figure 22

**FIGURE 23**

FIGURE 24

# Stability of output versus time

**FIGURE 25**

FIGURE 26

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 61419657 A
- US 508959 A
- US 7560076 B
- US 20090285731 A1
- US 20100104667 A1
- US 20100043788 A1
- US 20070089739 A
- US 8066904 B
- US 20060172018 A
- US 20090285731 A
- US 8057742 B
- US 7947227 B
- US 7914743 B
- US 20100043787 A
- US 20100043789 A
- US 20100043788 A
- US 20100030091 A
- US 20100089392 A

- US 20100104667 A
- US 20110220103 A
- US 20110240019 A
- US 201102362335 A
- US 201100259325 A
- US 7618594 B
- US 094541 A
- US 29360522 B
- US 29360525 B
- US 20110220103 A1
- US 12541144 B
- US 094535 A
- US 20110259325 A
- US 61263332 A
- US 61300425 B
- US 7520866 B
- US 7192018 B
- US 6793644 B

**Non-patent literature cited in the description**

- Nitric oxide (NO) and obstructive sleep apnea (OSA). *Sleep Breath,* June 2003, vol. 7 (2), 53-62
- Circulating Nitric Oxide Is Suppressed in Obstructive Sleep Apnea and Is Reversed by Nasal Continuous Positive Airway Pressure. *Am. J. Respir. Crit. Care Med.,* December 2000, vol. 162 (6), 2166-2171
- Outpatient Inhaled Nitric Oxide in a Patient with Idiopathic Pulmonary Fibrosis: A Bridge to Lung Transplantation. *Journal of Heart Lung Transplantation,* 2001, vol. 20, 1224-1227
- **CHANNICK, R. N. ; J. W. NEWHART et al.** Pulsed delivery of inhaled nitric oxide to patients with primary pulmonary hypertension: an ambulatory delivery system and initial clinical tests. *Chest,* 1996, vol. 109 (6), 1545-1549
- **PEREZ-PENATE, G. M. ; G. JULIA-SERDA et al.** Long-term inhaled nitric oxide plus phosphodiesterase 5 inhibitors for severe pulmonary hypertension. *J Heart Lung Transplant,* 2008, vol. 27 (12), 1326-1332
- **KUMAR ASHUTOSH ; KISHOR PHADKE ; JODY FRAGALE JACKSON ; DAVID STEELE.** Use of nitric oxide inhalation in chronic obstructive pulmonary disease. *Thorax,* 2000, vol. 55, 109-113

- **K VONBANK ; R ZIESCHE ; T W HIGENBOTTAM ; L STIEBELLEHNER ; V PETKOV ; P SCHENK ; P GERMANN ; L H BLOCK.** Controlled prospective randomised trial on the effects on pulmonary haemodynamics of the ambulatory long term use of nitric oxide and oxygen in patients with severe COPD. *Thorax,* 2003, vol. 58, 289-293
- **YOSHIDA et al.** The effect of low-dose inhalation of nitric oxide in patients with pulmonary fibrosis. *Eur Respir J,* 1997, vol. 10, 2051-4
- **GHOFRANI et al.** Sildenafil for treatment of lung fibrosis and pulmonary hypertension: a randomised controlled trial. *Lancet,* 2002, vol. 360, 895-900
- Platelet Activation in Patients with Sickle Cell Disease, Hemolysis-Associated Pulmonary Hypertension, and Nitric Oxide by Cell-Free Hemoglobin. *Blood,* 15 September 2007, vol. 110 (6), 2166-72
- Patterns of Arginine and Nitric Oxide in Patients with Sickle Cell Disease with Vaso-Occlusive Crisis and Acute Chest Syndrome. *Journal of Pediatric Hematology/Oncology,* December 2000, vol. 22 (6), 515-20

- Chronic Sickle Cell Lung Disease: New Insights into the Diagnosis, Pathogenesis and Treatment of Pulmonary Hypertension British. *Journal of Hematology,* 2005, vol. 129, 449-464
- Preliminary Assessment of Inhaled Nitric Oxide for Acute Vaso-occlusive Crisis in Pediatric Patients with Sickle Cell Disease. *JAMA,* 2003, vol. 289, 1136-1142
- Nitric Oxide Successfully Used to Treat Acute Chest Syndrome of Sickle Cell Disease in a Young Adolescent. *Critical Care Medicine,* November 1999, vol. 27 (11), 2563-8
- Nitric oxide deficiency in chronic kidney disease. *Am J Physiol Renal Physiol,* 2008, vol. 294, F1-F9
- What is the role of nitric oxide in murine and human host defense against tuberculosis?. *Am. J. Respir. Cell. Mol. Biol.,* vol. 25, 606-612
- *The Proceedings of the American Thoracic Society,* 2006, vol. 3, 161-165
- *Inhibition of Respiration by Nitric Oxide Induces a Mycobacterium tuberculosis Dormancy Program Nitric Oxide,* February 2006, vol. 14 (1), 21-9
- *Inhaled Nitric Oxide Treatment of Patients with Pulmonary Tuberculosis Evidenced by Positive Sputum Smears Antimicrobial Agents and Chemotherapy,* March 2005, vol. 49 (3), 1209-1212
- Nitric oxide inhibits interferon regulatory factor-1 and nuclear factor-kB in rhinovirus infected epithelial cells. *J. Allergy Clin. Immunol,* 2009
- Role of nasal nitric oxide in the resolution of experimental rhinovirus infection. *J. Allergy Clin. Immunol.,* 2004, vol. 113, 697-702
- Critically ill patients with 2009 influenza A(H1N1) infection in Canada. *JAMA,* 04 November 2009, vol. 302 (17), 1872-9
- Effects of Inhaled Nitric Oxide in the Treatment of Acute Hypoxemic Respiratory Failure (AHRF) in Pediatrics ClinicalTrials.gov Identifier: NCT00041561-Phase II. *Pediatric Acute Hypoxemic Respiratory Failure: Management of Oxygenation J Intensive Care Med,* 2004, vol. 19, 140
- Inhaled NO for Preterm Infants-Getting to Yes?. *N Engl J Med,* 27 July 2006, vol. 355 (4), 404-406
- Inhaled Nitric Oxide in Preterm Infants Undergoing Mechanical Ventilation. *N Engl J Med.,* 27 July 2006, vol. 355 (4), 343-54
- Early Inhaled Nitric Oxide Therapy in Premature Newborns with Respiratory Failure. *N Engl J Med.,* 27 July 2006, vol. 355 (4), 354-64
- Preemie Inhaled Nitric Oxide Study. Inhaled nitric oxide for premature infants with severe respiratory failure. *N Engl J Med.,* 07 July 2005, vol. 353 (1), 13-22
- Inhaled Nitric Oxide in Premature Infants with the Respiratory Distress Syndrome. *N Engl J Med,* 2003, vol. 349, 2099-107
- Neuro-developmental Outcomes of Premature Infants Treated with Inhaled Nitric Oxide. *N Engl J Med,* 07 July 2005, vol. 353 (1), 23-32
- Beneficial effects of inhaled nitric oxide in adult cardiac surgical patients. *Ann Thorac Surg,* 2002, vol. 73, 529-533
- Treatment of pulmonary hypertension in patients undergoing cardiac surgery with cardiopulmonary bypass: a randomized, prospective, double-blind study. *J Cardiovasc Med (Hagerstown),* February 2006, vol. 7 (2), 119-23
- Response to nitric oxide during adult cardiac surgery. *Journal of Investigative Surgery,* 2002, vol. 15 (1), 5-14
- Dose response to nitric oxide in adult cardiac surgery patients. *Journal of Clinical Anesthesia,* 2001, vol. 13 (4), 281-60
- **MINAMISHIMA.** *Am. J. Physiol. Heart Circ. Physiol.,* April 2011, vol. 300, H1477-H1483
- Inhaled NO accelerates restoration of liver function in adults following orthotopic liver transplantation. *J Clin Invest.,* September 2007, vol. 117 (9), 2583-91
- Right ventricular failure after left ventricular assist device insertion: preoperative risk factors. *Interact CardioVasc Thorac Surg,* 2006, vol. 5, 379-382
- Randomized, double-blind trial of inhaled nitric oxide in LVAD recipients with pulmonary hypertension. *Ann Thorac Surg.,* 1998, vol. 65, 340-345
- Hemodynamic effects of inhaled nitric oxide in right ventricular myocardial infarction and cardiogenic shock. *J Am Coll Cardiol,* 2004, vol. 44, 793-8
- *The Role of Nitric Oxide and Vasopressin in Refractory Right Heart Failure Journal of Cardiovascular Pharmacology and Therapeutics,* 2004, vol. 9 (1), 9-11
- 2006 national hospital discharge survey. *Natl Health Stat Rep No.5, www.cdc.gov/nchs/data/nhsr/nhsr005*
- Nitric oxide mechanisms of protection in ischemia and reperfusion injury. *J Invest Surg,* vol. 22, 46-55
- Hemodynamic effects of inhaled nitric oxide in right ventricular myocardial infarction and cardiogenic shock. *J Am Coll Cardiol,* vol. 44, 793-798
- Inhaled NO as a Therapeutic Agent. *Cardiovascular Research,* 07 May 2007, vol. 75, 339-48
- The Influence of Inhaled Nitric Oxide on Cerebral Blood Flow and Metabolism in a Child with Traumatic Brain Injury. *Anesth Analg,* 2001, vol. 93, 351-3
- The beneficial effects of inhaled nitric oxide in patients with severe traumatic brain injury complicated by acute respiratory distress syndrome: a hypothesis. *Journal of Trauma Management & Outcomes,* 2008, vol. 2, 1

- Successful Use Of Inhaled Nitric Oxide To Decrease Intracranial Pressure In A Patient With Severe Traumatic Brain Injury Complicated By Acute Respiratory Distress Syndrome: A Role For An Anti-Inflammatory Mechanism?. *Scandinavian Journal of Trauma, Resuscitation and Emergency Medicine,* 2009, vol. 17, 5
- Inhaled Nitric Oxide Improves Pulmonary Functions Following Massive Pulmonary Embolism: A Report of Four Patients and Review of the Literature. *Lung,* 2006, vol. 184, 1-5
- Airway Nitric Oxide in Patients With Cystic Fibrosis Is Associated With Pancreatic Function. *Pseudomonas Infection, and Polyunsaturated Fatty Acids, Chest,* 2007, vol. 131, 1857-1864
- **BLACK, D.** Has malaria met its match?. *Toronto Star,* 10 October 2011
- Treatment of Acute Mountain Sickness and High Altitude Pulmonary Edema. *MJAFI,* 2004, vol. 60, 384-38
- Randomized, Controlled Trial of Regular Sildenafil Citrate in the Prevention of Altitude Illness NCT00627965; Inhaled NO and high altitude. *N. Engl. J. Med.,* 1996, vol. 334, 624-9
- Effects of Inhaled Nitric Oxide and Oxygen in High-Altitude Pulmonary Edema. *Circulation,* 1998, vol. 98, 2441-2445
- *Treatment of Acute Mountain Sickness and High Altitude Pulmonary Oedema MJAFI,* 2004, vol. 60, 384-387
- Acute Mountain Sickness, High Altitude Cerebral Oedema, High Altitude Pulmonary edema: The Current Concepts. *MJAFI,* 2008, vol. 64, 149-153
- High-altitude pulmonary oedema: still a place for controversy?. *Thorax,* 1995, vol. 50, 923-929